# FASCICULE DE BREVET EUROPEEN

(11) **EP 2 858 966 B1**
(45) Date de publication et mention de la délivrance du brevet: **10.08.2016**
(21) Numéro de dépôt: 13728711.6
(22) Date de dépôt: 06.06.2013
(51) Int. Cl.: C07B 37/04, C07C 37/20, C07C 39/21, C07C 45/68, C07C 49/784, C07C 49/813, C07C 49/84, C07C 213/06, C07C 213/08, C07C 49/83, C07C 217/18, C07C 213/04

(54) **PROCEDE DE CREATION DE LIAISONS CARBONE-CARBONE A PARTIR DE COMPOSES CARBONYLES**
VERFAHREN ZUR ERZEUGUNG VON KOHLENSTOFF-KOHLENSTOFF-VERBINDUNGEN UNTER VERWENDUNG VON CARBONYLVERBINDUNGEN
PROCESS FOR CREATING CARBON-CARBON BONDS USING CARBONYL COMPOUNDS

(30) Priorité: 06.06.2012 FR 1255275
(43) Date de publication de la demande: 15.04.2015
(73) Titulaire: Centre National de la Recherche Scientifique (CNRS), 75016 Paris (FR); Université Montpellier 2, Sciences et Techniques, 34095 Montpellier Cedex 5 (FR); Ecole Nationale Supérieure de Chimie de Montpellier, 34296 Montpellier (FR)
(72) Inventeur: TAILLEFER, Marc, F-34570 Vailhauques (FR); MONNIER, Florian, F-34070 Montpellier (FR); TLILI, Anis, 69100 Villeurbanne (FR); DANOUN, Grégory, 28210 Villemeux sur eure (FR)
(74) Mandataire: Grosbois, Mathilde
(86) Numéro de dépôt international: PCT/EP2013/061697
(87) Numéro de publication internationale: WO 2013/182640

(56) Documents cités:
- GUEDIRA ET AL: "Ambident behavior of ketone enolate anions in SNAr substitutions on fluorobenzonitrile substrates", JOURNAL OF ORGANIC CHEMISTRY, ACS, US, vol. 57, no. 21, 1 janvier 1992 (1992-01-01), pages 5577-5585, XP002105274, ISSN: 0022-3263, DOI: 10.1021/JO00047A008
- EDUARDAS SKUCAS ET AL: "Enantioselective [alpha]-Vinylation of Aldehydes via the Synergistic Combination of Copper and Amine Catalysis", JOURNAL OF THE AMERICAN CHEMICAL SOCIETY, vol. 134, no. 22, 22 mai 2012 (2012-05-22) , pages 9090-9093, XP055051806, ISSN: 0002-7863, DOI: 10.1021/ja303116v
- CHEN L ET AL: "Synthesis of multisubstituted furans via copper-catalyzed intramolecular O-vinylation of ketones with vinyl bromides", TETRAHEDRON LETTERS, ELSEVIER, AMSTERDAM, NL, vol. 51, no. 28, 14 juillet 2010 (2010-07-14), pages 3678-3681, XP027080099, ISSN: 0040-4039, DOI: 10.1016/J.TETLET.2010.05.040 [extrait le 2010-05-15]

## Description

La présente invention concerne un procédé de création de liaisons carbone-carbone (liaison C-C) à partir d'un composé carbonylé, et notamment un procédé d'arylation de composés carbonylés. L'invention concerne également la préparation de synthons (ou « building blocks ») permettant la préparation de molécules d'intérêt, notamment dans le domaine de la pharmacie, des cosmétiques, de l'agrochimie, etc.

La préparation de nombreuses familles de molécules actives, notamment dans le domaine de la pharmacie ou de l'agrochimie, requiert la formation de liaisons carbone-carbone (liaison C-C) entre un composé saturé et un composé de type carbonylé. La création de telles liaisons carbone-carbone peut notamment être réalisée par arylation de composés carbonylés, notamment par arylation en alpha du carbonyle du composé carbonylé. De manière habituelle, toute molécule résultant de l'arylation de composés carbonylés est obtenue par catalyse au palladium mettant en oeuvre des ligands complexes de type phosphine (Bellina et al. Chem.Rev., 2010, 110, 1082-1146 et Johansson et al., Angew.Chem.Int., 2010, 49, 676-707).

Cependant, les catalyseurs à base de palladium ainsi que les ligands utilisés dans ces méthodes sont très couteux et toxiques. Il est donc nécessaire de fournir un procédé de création de liaisons C-C à partir d'un composé carbonylé, et notamment un procédé d'arylation de nucléophile de type carbonylé, permettant de répondre aux inconvénients des méthodes de l'état de la technique, et notamment qui soit économique et qui présente une toxicité moindre voire nulle.

Il est également connu :
- de Guedira et al. (Journal of Pharmaceutical Sciences, 1993, 82(9), 927-933) des réactions de substitution nucléophile aromatique. Cependant, les rendements et sélectivités obtenus sont faibles ;
- de Skucas et al. (JACS, 2012, 134, 9090-9093) un procédé d'α-vinylation d'aldéhyde mettant en oeuvre une combinaison synergique de cuivre et d'un catalyseur de type amine ;
- de Chen et al. (Tetrahedron Letters, 2010, 51, 3678-3681) des réactions intramoléculaires de type O-vinylation.

Un objectif de la présente invention est donc de fournir un procédé de création de liaisons C-C à partir de composés carbonylés qui soit économique et qui présente une toxicité moindre voire nulle.

Un objectif particulier de l'invention est de fournir un tel procédé qui soit un procédé d'arylation de composé carbonylé.

Un autre objectif de la présente invention est de fournir un procédé de préparation de synthons pour la préparation de molécules d'intérêt notamment dans le domaine de la pharmacie, de l'agrochimie, etc.

Un autre objectif de la présente invention est de fournir un procédé de préparation de molécules d'intérêt, notamment dans le domaine de la pharmacie, de l'agrochimie, etc.

D'autres objectifs encore apparaitront à la lecture de la description de l'invention qui suit.

La présente invention concerne un procédé de préparation d'un composé de formule (I) par réaction entre un composé de formule (II) et un composé de formule (III) en présence d'un catalyseur comprenant du cuivre, d'un ligand et d'une base 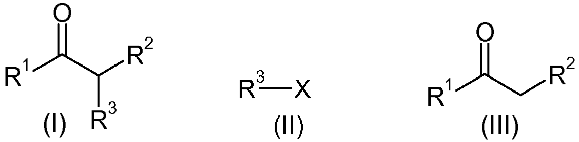 dans lesquelles :
- R¹ représente :
   - un atome d'hydrogène ;
   - un groupe alkyle comprenant de 1 à 15 atomes de carbone, linéaire ou ramifié,
   - un groupe alcényle comprenant de 1 à 15 atomes de carbone, linéaire ou ramifié, et comprenant au moins une double liaison;
   - un groupe aryle comprenant de 6 à 10 atomes de carbone, substitué ou non substitué ;
   - un groupe hétéroaryle comprenant de 5 à 10 membres dont au moins un hétéroatome, notamment choisi parmi l'atome d'azote ou l'atome d'oxygène, substitué ou non substitué ;
- R² représente :
   - un groupe alkyle comprenant de 1 à 15 atomes de carbone, linéaire ou ramifié ;
   - un groupe alcényle comprenant de 1 à 15 atomes de carbone, linéaire ou ramifié, et comprenant au moins une double liaison ;
   - un groupe aryle comprenant de 6 à 10 atomes de carbone, substitué ou non substitué ;
   - un groupe hétéroaryle comprenant de 5 à 10 membres dont au moins un hétéroatome, notamment choisi parmi l'atome d'azote ou l'atome d'oxygène, substitué ou non substitué ;
- R³ représente :
   - un groupe vinyle, substitué ou non substitué ;
   - un groupe aryle comprenant de 6 à 10 atomes de carbone, substitué ou non substitué ;
   - un groupe hétéroaryle comprenant de 5 à 10 membres dont au moins un hétéroatome, notamment choisi parmi l'atome d'azote ou l'atome d'oxygène, substitué ou non substitué ;
- X représente un atome d'halogène choisi parmi le fluor, le chlore, le brome ou l'iode ; un tosylate ou un mésylate
De préférence X représente un atome d'halogène choisi parmi le fluor, le chlore, le brome ou l'iode.

Dans un mode de réalisation, le composé de formule (II) est un composé de formule (IIa) 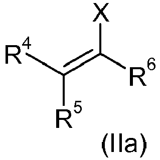 dans laquelle :
- X est tel que défini pour le composé de formule (II), de préférence X représente un atome d'halogène choisi parmi le fluor, le chlore, le brome ou l'iode, de préférence X est l'iode ou le brome ;
- R⁴, R⁵ et R⁶, identiques ou différents, représentent :
   - un atome d'hydrogène ;
   - un groupe alkyle comprenant de 1 à 15 atomes de carbone, linéaire ou ramifié, pouvant comprendre une ou plusieurs insaturations ;
   - un groupe aryle comprenant de 6 à 10 atomes de carbone, substitué ou non substitué ;
   - un atome d'halogène choisi parmi le chlore, le brome, le fluor et l'iode.

Dans un autre mode de réalisation, R³ est un hétéroaryle comprenant de 5 à 10 membres dont au moins un hétéroatome choisi parmi un atome d'azote ou un atome d'oxygène. L'hétéroaryle peut être substitué notamment par un ou plusieurs substituants choisis parmi :
- un groupe de formule -(O)ₙalkyle comprenant de 1 à 15 atomes de carbone, linéaire ou ramifié, de préférence de 1 à 10 atomes de carbone, par exemple de 1 à 6 atomes de carbone, éventuellement substitué par un ou plusieurs atome d'halogène, de préférence de fluor ; et n représente 0 ou 1 ;
- un groupe alcényle comprenant de 1 à 15 atomes de carbone, linéaire ou ramifié, et comprenant au moins une double liaison ;
- un atome d'halogène, notamment choisi parmi le chlore, le fluor, le brome ou l'iode, de préférence le fluor ou le chlore ;
- un groupe CN ; ou
- un groupe NH₂.
De préférence les substituants de l'hétéroaryle sont choisis parmi :
- un groupe de formule -(O)ₙalkyle comprenant de 1 à 15 atomes de carbone, linéaire ou ramifié, de préférence de 1 à 10 atomes de carbone, par exemple de 1 à 6 atomes de carbone, éventuellement substitué par un ou plusieurs atome d'halogène, de préférence de fluor ; et n représente 0 ou 1 ;
- un groupe alcényle comprenant de 1 à 15 atomes de carbone, linéaire ou ramifié, et comprenant au moins une double liaison ;
- un atome d'halogène, notamment choisi parmi le chlore, le fluor, le brome ou l'iode, de préférence le fluor ou le chlore ;
Dans ce mode de réalisation particulier, de préférence X représente un atome d'halogène choisi parmi le fluor, le chlore, le brome ou l'iode, de préférence X est l'iode.

Dans un mode de réalisation préféré, R³ représente un aryle, substitué ou non substitué, comprenant de 6 à 10 atomes de carbone. L'aryle peut être substitué par un ou plusieurs substituants choisis parmi:
- un groupe de formule -(O)ₙalkyle comprenant de 1 à 15 atomes de carbone, linéaire ou ramifié, de préférence de 1 à 10 atomes de carbone, par exemple de 1 à 6 atomes de carbone, éventuellement substitué par un ou plusieurs atome d'halogène, de préférence de fluor ; et n représente 0 ou 1 ;
- un groupe alcényle comprenant de 1 à 15 atomes de carbone, linéaire ou ramifié, et comprenant au moins une double liaison;
- un atome d'halogène, notamment choisi parmi le chlore, le fluor, le brome ou l'iode, de préférence le fluor ou le chlore ;
- un groupe de formule CN ;
- un groupe de formule NH₂ ;
- un groupe aryle comprenant de 6 à 10 atomes de carbone, substitué ou non substitué ;
- un groupe de formule (CH₂)ₘ-aryle dans laquelle m représente un nombre entier allant de 1 à 10, de préférence de 1 à 5, et l'aryle, substitué ou non substitué, comprend de 6 à 10 atomes de carbone.

De préférence l'aryle peut être substitué par un ou plusieurs substituants choisis parmi :
- un groupe de formule -(O)ₙalkyle comprenant de 1 à 10 atomes de carbone, linéaire ou ramifié, par exemple de 1 à 6 atomes de carbone et n représente 0 ou 1 ;
- groupe trifluoroalkyle comprenant de 1 à 10 atomes de carbone, linéaire ou ramifié, de préférence trifluorométhyle ;
- un atome de fluor ;
- un atome de chlore.
De préférence, R³ représente un phényle substitué ou non substitué.
Dans ce mode de réalisation, de préférence X représente un atome d'halogène choisi parmi le fluor, le chlore, le brome ou l'iode, de préférence X l'iode.

De préférence, dans les composés de formule (I) et (III) selon l'invention R¹ est choisi parmi :
- un atome d'hydrogène ;
- un groupe alkyle comprenant de 1 à 15 atomes de carbone, linéaire ou ramifié ;
- un groupe alcényle comprenant de 1 à 15 atomes de carbone, linéaire ou ramifié, et comprenant au moins une double liaison;
- un groupe aryle comprenant de 6 à 10 atomes de carbone, substitué ou non substitué, notamment par un ou plusieurs substituants choisis parmi :
   ▪ un atome d'halogène choisi parmi le chlore, le brome, le fluor ou l'iode ; de préférence le chlore, le brome ou le fluor, de préférence le chlore;
   ▪ un groupe hydroxy ;
   ▪ un groupe de formule -(O)ₚalkyle comprenant de 1 à 15 atomes de carbone, linéaire ou ramifié de préférence de 1 à 10 atomes de carbone, par exemple de 1 à 6 atomes de carbone et p représente 0 ou 1 ;
   ▪ un groupe alcényle comprenant de 1 à 15 atomes de carbone, linéaire ou ramifié, et comprenant au moins une double liaison;
   ▪ un groupe trifluoroalkyle comprenant de 1 à 10 atomes de carbones, linéaire ou ramifié, de préférence trifluorométhyle.

De préférence, dans les composés de formule (I) et (III) selon l'invention R¹ représente un groupe alkyle comprenant de 1 à 15 atomes de carbone, linéaire ou ramifié; ou un groupe aryle, de préférence phényle, comprenant de 6 à 10 atomes de carbone, substitué ou non substitué, notamment par un ou plusieurs substituants choisis parmi:
▪ un atome d'halogène choisi parmi le chlore, le brome, le fluor ou l'iode ; de préférence le chlore, le brome ou le fluor, de préférence le chlore ;
▪ un groupe hydroxy ;
▪ un groupe de formule -(O)ₚalkyle comprenant de 1 à 15 atomes de carbone, linéaire ou ramifié, de préférence de 1 à 10 atomes de carbone, par exemple de 1 à 6 atomes de carbone et p représente 0 ou 1 ;
▪ un groupe trifluoroalkyle comprenant de 1 à 10 atomes de carbones, linéaire ou ramifié, de préférence trifluorométhyle.

De préférence, dans les composés de formule (I) et (III) selon l'invention R¹ représente un groupe aryle comprenant de 6 à 10 atomes de carbone, substitué ou non substitué, notamment par un ou plusieurs substituants choisis parmi :
▪ un atome d'halogène choisi parmi le chlore, le brome, le fluor ou l'iode ; de préférence le chlore, le brome ou le fluor, de préférence le chlore ;
▪ un groupe hydroxy ;
▪ un groupe de formule -(O)ₚalkyle comprenant de 1 à 15 atomes de carbone, linéaire ou ramifié, de préférence de 1 à 10 atomes de carbone, par exemple de 1 à 6 atomes de carbone et p représente 0 ou 1 ;
▪ un groupe trifluoroalkyle comprenant de 1 à 10 atomes de carbones, linéaire ou ramifié, de préférence trifluorométhyle.

De préférence, dans les composés de formule (I) et (III) selon l'invention R¹ représente un phényle, substitué ou non substitué, notamment par un ou plusieurs substituants choisis parmi :
▪ un atome d'halogène choisi parmi le chlore, le brome, le fluor ou l'iode ; de préférence le chlore, le brome ou le fluor, de préférence le chlore ;
▪ un groupe hydroxy ;
▪ un groupe de formule -(O)ₚalkyle comprenant de 1 à 15 atomes de carbone, linéaire ou ramifié, de préférence de 1 à 10 atomes de carbone, par exemple de 1 à 6 atomes de carbone et p représente 0 ou 1 ;
▪ un groupe trifluoroalkyle comprenant de 1 à 5 atomes de carbones, linéaire ou ramifié, de préférence trifluorométhyle.

De préférence, dans les composés de formule (I) et (III) selon l'invention R² est choisi parmi :
- un groupe alkyle comprenant de 1 à 15 atomes de carbone, linéaire ou ramifié;
- un groupe alcényle comprenant de 1 à 15 atomes de carbone, linéaire ou ramifié, et comprenant au moins une double liaison;
- un groupe aryle comprenant de 6 à 10 atomes de carbone, substitué ou non substitué, notamment par un ou plusieurs substituants choisis parmi :
   ▪ un atome d'halogène choisi parmi le chlore, le brome, le fluor ou l'iode ; de préférence le chlore, le brome ou le fluor ;
   ▪ un groupe de formule -(O)_{q}alkyle comprenant de 1 à 15 atomes de carbone, linéaire ou ramifié, de préférence de 1 à 10 atomes de carbone, par exemple de 1 à 6 atomes de carbone et q représente 0 ou 1 ;
   ▪ un groupe hydroxy ;
   ▪ un groupe alcényle comprenant de 1 à 15 atomes de carbone, linéaire ou ramifié, et comprenant au moins une double liaison ;
   ▪ un groupe trifluoroalkyle comprenant de 1 à 10 atomes de carbones, linéaire ou ramifié, de préférence trifluorométhyle.

De préférence, dans les composés de formule (I) et (III) selon l'invention R² représente un groupe aryle comprenant de 6 à 10 atomes de carbone, substitué ou non substitué, notamment par un ou plusieurs substituants choisis parmi :
▪ un atome d'halogène choisi parmi le chlore, le brome, le fluor ou l'iode ; de préférence le chlore, le brome ou le fluor ;
▪ un groupe de formule -(O)_{q}alkyle comprenant de 1 à 15 atomes de carbone, linéaire ou ramifié, de préférence de 1 à 10 atomes de carbone, par exemple de 1 à 6 atomes de carbone et q représente 0 ou 1 ;
▪ un groupe hydroxy ;
▪ un groupe trifluoroalkyle comprenant de 1 à 10 atomes de carbones, linéaire ou ramifié, de préférence trifluorométhyle.

De préférence, dans les composés de formule (I) et (III) selon l'invention R² représente un phényle, substitué ou non substitué, notamment par un ou plusieurs substituants choisis parmi :
▪ un atome d'halogène choisi parmi le chlore, le brome, le fluor ou l'iode ; de préférence le chlore, le brome ou le fluor ;
▪ un groupe hydroxy ;
▪ un groupe de formule -(O)_{q}alkyle comprenant de 1 à 15 atomes de carbone, linéaire ou ramifié, de préférence de 1 à 10 atomes de carbone, par exemple de 1 à 6 atomes de carbone et q représente 0 ou 1 ;
▪ un groupe trifluoroalkyle comprenant de 1 à 5 atomes de carbones, linéaire ou ramifié, de préférence trifluorométhyle.

De préférence, dans le composé de formule (II), X représente l'iode.

Dans le cadre de l'invention, on entend par :
- un atome d'halogène, un atome de fluor, chlore, brome ou iode ;
- un groupe alkyle, un groupe aliphatique saturé, linéaire ou ramifié comprenant de 1 à 15 atomes de carbone. Par exemple on peut citer, méthyle, éthyle, propyle, butyle, ter-butyle...
- un groupe alcényle, un groupe aliphatique mono ou polyinsaturé, linéaire ou ramifié, comprenant de 1 à 15 atomes de carbone, par exemple comprenant 1 ou 2 insaturations éthylénique ;
- un groupe aryle, un groupe cyclique aromatique comprenant de 6 à 10 atomes de carbone ; ledit cycle pouvant être éventuellement substitué ;
- un groupe hétéroaryle, un groupe cyclique aromatique comprenant de 5 à 10 membres dont au moins un hétéroatome notamment choisi parmi l'azote, l'oxygène et le soufre, de préférence l'azote ou l'oxygène ;
- un groupe hydroxy, un groupe de formule OH.

Le catalyseur utilisé dans l'invention est notamment choisi parmi le cuivre métallique, les oxydes de cuivre (I) ou de cuivre (II), les hydroxydes de cuivre (I) ou de cuivre (II), les sels inorganiques ou organiques de cuivre (I) ou de cuivre (II) et les complexes de cuivre (I) ou de cuivre (II) avec des ligands usuels, ou leurs mélanges.

Des exemples préférés de catalyseur comprenant du cuivre incluent, sans en être limité, le cuivre (0), les halogénures de cuivre (exemple : iodures de cuivre (I), bromure de cuivre (I), bromure de cuivre (II), chlorure de cuivre (I), chlorure de cuivre (II)), les oxydes ou hydroxydes de cuivre (exemple : oxyde de cuivre (I) , oxyde de cuivre(II), hydroxydes de cuivre (II)), les nitrates de cuivre (exemple : nitrate de cuivre (I), nitrates de cuivre (II)), les sulfates ou sulfites de cuivre (exemple : sulfate de cuivre (I) , sulfate de cuivre (II) sulfite de cuivre (I)) les sels organiques de cuivre dans lesquelles le contre ions présente au moins un atome de carbone (exemple : carbonate de cuivre (II) acétate de cuivre (I) acétate de cuivre (II), trifluorométhylsulfonate de cuivre (II), méthylate de cuivre (I), méthylate de cuivre (II), acétylacétonate de cuivre(II)), les cyanures de cuivre (CuCN), ou leur mélange.

Des catalyseurs préférés comprenant du cuivre sont le cuivre (0), l'iodure de cuivre (I) (Cul), l'oxyde de cuivre (II) (Cu₂O), l'acétylacétonate de cuivre (II) [Cu(acac)₂], Cul + Cu(acac)₂, ou leur mélange.

De préférence, le catalyseur comprenant du cuivre est l'iodure de cuivre Cul.

Dans un mode de réalisation le procédé de l'invention est mis en oeuvre en présence d'un solvant.

Le solvant est alors choisi dans le groupe constitué par l'eau, les solvants organiques et leurs mélanges, de préférence les solvants organiques et leurs mélanges ainsi que leurs mélanges avec l'eau.

Dans un mode de réalisation le solvant organique peut être choisi parmi :
- les carboxamides linéaires ou cycliques, de préférence, N-diméthylacetamide (DMA), N,N-diéthylacetamide, diméthylformamide (DMF), diéthylformamide ou le 1-méthyl-2-pyrrolidinone (NMP) ;
- le diméthylsulfoxide (DMSO) ;
- l'hexaméthylphosphotriamide (HMPT) ;
- le tetraméthylurea ;
- le benzène ;
- les composés à base nitro de préférence nitrométhane, nitroéthane, 1-nitropropane, 2-nitropropane ou nitrobenzene;
- les nitriles aliphatiques ou aromatiques de préférence acétonitrile, propionitrile, butanenitrile, isobutanenitrile, pentanenitrile, 2-methylglutaronitrile ou adiponitrile ;
- le tétraméthylène sulfone ;
- les carbonates organiques de préférence diméthylcarbonate, düsopropylcarbonate ou di-n-butylcarbonate ;
- les esters alkylés de préférence éthyl acétate ou isopropyl acétate ;
- les éthers aliphatiques ou aromatiques de préférence 1,4-dioxane ;
- les composés hydrocarbonés halogénés ou non halogénés de préférence toluène ou chlorobenzène ;
- les cétones de preference acétone, methyléthylcétone, methylisobutylcétone (MIBK), cyclopentanone, cyclohexanone;
- les hétérocycles comprenant un groupement nitrogène de préférence pyridine, picoline ou quinolines ;
- les alcools à l'exception de l'éthanol, de préférence le tert-butanol seuls ou en mélange.

Les solvants préférés de l'invention sont le dioxane, le DMF, le DMA, l'acétonitrile et le tert-butanol. De préférence, les solvants sont le dioxane et le tert-butanol.

Selon l'invention, de nombreux types de ligand peuvent convenir, notamment les ligands bidentates, notamment choisi parmi :
- les dicétones, notamment de formule (i) 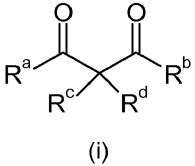 dans laquelle :
   ▪ R^{a} et R^{b}, identiques ou différents, représentent une chaîne alkyle, linéaire ou ramifiée en C₁ à C₁₀, de préférence C₁ à C₄, par exemple t-butyle, méthyle, ou un radical aryle en C₆ à C₁₀, notamment phényle ;
   ▪ R^{c} et R^{d}, représentent un atome d'hydrogène ; ou
   ▪ R^{a} ou R^{b}, forme avec le groupe C(O) qui les porte et avec l'un de R^{c} ou R^{d} un carbocycle à 6 membres ; ou
- les diamines notamment de formule (ii)

   NR^{e}R^{f}-(CH₂)ᵣ-NR^{g}R^{h} (ii)

   dans laquelle :
   ▪ R^{e}, R^{f}, R^{g}, R^{h}, identiques ou différents, représentent un atome d'hydrogène ou un groupe alkyle en C₁ à C₁₀;
   ▪ r représente un nombre entier compris entre 1 et 10, de préférence entre 1 et 5 ; ou
- les composés polycycliques aromatiques comprenant au moins deux hétéroatomes, notamment azote, notamment des tricycles, éventuellement substitué par un ou plusieurs groupes alkyles en C₁ à C₁₀, groupe NO₂, groupe aryle comprenant de 6 à 10 atomes de carbone, de préférence le composé polycyclique est la phénantroline, substituée ou non substituée.
De façon avantageuse le ligand peut être choisi parmi les groupes de formule : 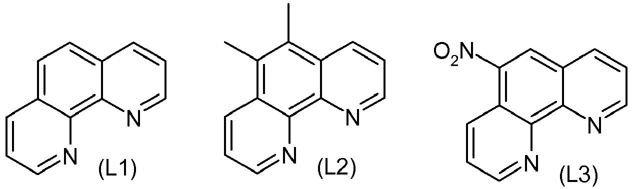 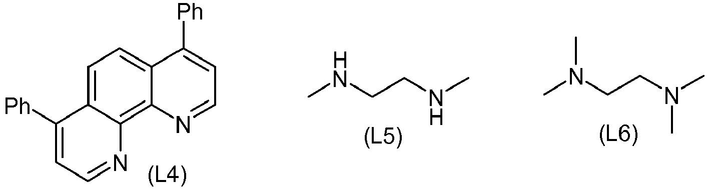 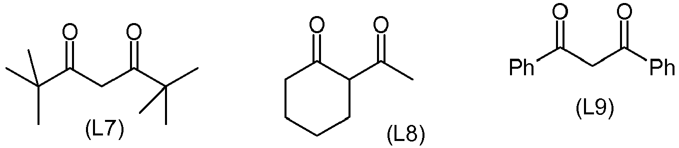 avec Ph représente un phényle.

De façon avantageuse le ligand est choisi parmi (L1), (L4) ou (L8).

De façon avantageuse :
- le solvant est le dioxane et le ligand est choisi parmi (L1), (L4), (L5), (L6), (L7), (L8) ou (L9), de préférence (L1), (L4) ; ou
- le solvant est le tert-butanol et le ligand est choisi parmi (L1), (L2), (L3), (L4), (L7) ou (L8), de préférence (L1), (L2), (L3), (L4) ou (L8).

Selon l'invention, la base peut être choisie parmi les alcoolates alcalins ou alcalino-terreux de formule (M¹(OR)ₛ) et/ou les phosphates alcalins ou alcalino-terreux de formule (M¹ₜ(OH)ᵤ(PO₄)ᵥ) et/ou les carbonates alcalins ou alcalino-terreux de formule (M'_{w}(CO₃)_{y}), ou leurs mélanges.

Selon l'invention la base peut être un alcoolate alcalin ou alcalino-terreux de formule (M¹ (OR)ₛ) dans laquelle M¹ représente Li, Na, K, Rb, Cs, Fr, Be, Mg, Ca, Sr, Ba ou Ra, de préférence Na ; s représente 1 ou 2 et R est choisi dans le groupe constitué par les groupes alkyle, benzyle et aryle ; notamment la base peut être le terbutylate de potassium.

Selon l'invention la base peut être un phosphate alcalin ou alcalino-terreux de formule (M¹ₜ(OH)ᵤ(PO₄)ᵥ) dans laquelle M¹ représente Li, Na, K, Rb, Cs, Fr, Be, Mg, Ca, Sr, Ba ou Ra, de préférence K ; t représente un nombre entier allant de 1 à 10, de préférence de 1 à 5 ; u représente un nombre entier allant de 0 à 2 et v représente un nombre entier allant de 1 à 10.

Selon l'invention la base peut être un carbonate alcalin ou alcalino-terreux de formule (M¹_{w}(CO₃)_{y}) dans laquelle M¹ est choisi dans le groupe constitué par Li, Na, K, Rb, Cs, Fr, Be, Mg, Ca, Sr, Ba ou Ra, de préférence Cs ou K ; w représente un nombre entier allant de 1 à 10, de préférence w égale 2 , et y représente un nombre entier allant de 1 à 10, de préférence y égale 1 ; notamment la base peut être un carbonate de césium (Cs₂CO₃, un carbonate de potassium (K₂CO₃), ou un carbonate de rubidium (Rb₂CO₃).

De manière avantageuse, la base peut être choisie parmi Cs₂CO₃, K₂CO₃ et Rb₂CO₃.

De façon particulièrement avantageuse, le procédé de l'invention peut être mis en oeuvre à des températures plus basses que les procédés de l'état de la technique catalysés au palladium, ceci peut permettre une économie d'énergie.

Ainsi, le procédé de l'invention peut être mis en oeuvre à une température allant de 50 à 200°C, de préférence de 70 à 150°C.

De préférence, le procédé est mis en oeuvre à pression atmosphérique, à pression autogène du milieu ou avec une légère surpression.

De préférence, dans le procédé de l'invention, la quantité molaire de catalyseur comprenant du cuivre est de 0,001 à 100% par rapport au nombre de moles du composé de formule (II), de préférence de 0,001 à 50%, de manière plus préférée de 0,01 à 25%, par exemple de 1 à 20%.

De préférence, dans le procédé de l'invention le ratio molaire ligand/catalyseur comprenant du cuivre est de 0,5 à 1000, de préférence de 0,5 à 100, de préférence de 0,5 à 5, par exemple de 1 à 2.

De préférence, dans le procédé de l'invention, le ratio molaire composé de formule (III)/composé de formule (II) est de 0,5 à 10, de préférence de 0,5 à 5, notamment de 1,5 à 5..

De préférence, dans le procédé de l'invention, la quantité molaire de base est de 0,2 à 4 équivalents, de préférence de 0,5 à 2 équivalents, par rapport à la quantité molaire de composé de formule (II).

De manière habituelle, le temps de réaction est compris entre 1 et 36 heures, de préférence de 12 à 48 heures.

De manière avantageuse, le procédé de l'invention peut permettre la préparation de synthon (ou « building blocks ») pour la préparation de molécules d'intérêts, notamment dans le domaine de la pharmacie, de l'agrochimie, etc. Le procédé de l'invention ainsi mis en oeuvre présente l'avantage d'être compatible avec de nombreuses fonctions chimiques permettant ainsi de réduire, voir d'éviter, la mise en oeuvre de réactions de protection/déprotection.

Ainsi, l'invention concerne également un procédé de préparation de synthon pour la préparation de molécules d'intérêt comprenant les étapes suivantes :
1) mise en oeuvre du procédé de préparation d'un composé de formule (I) selon l'invention ;
2) fonctionnalisation du composé de formule (I) par des méthodes connues de l'homme du métier.

De préférence, la fonctionnalisation peut consister par exemple en :
- une réaction d'addition d'un réactif organométallique sur le carbonyle du composé de formule (I) ;
- l'addition d'un groupe alkyle, aryle, hétéroaryle ;
- une réaction d'oléfination ; ou
- une réaction de Shapiro.

De façon particulière, l'invention concerne également la préparation d'un composé de formule (IV) avec R représente un groupe hydroxy comprenant la mise en oeuvre du procédé de création de liaison carbone-carbone selon l'invention

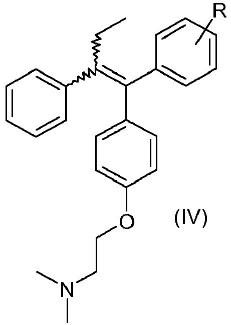

De préférence, les composés de formule (IV) sont les composés suivants : 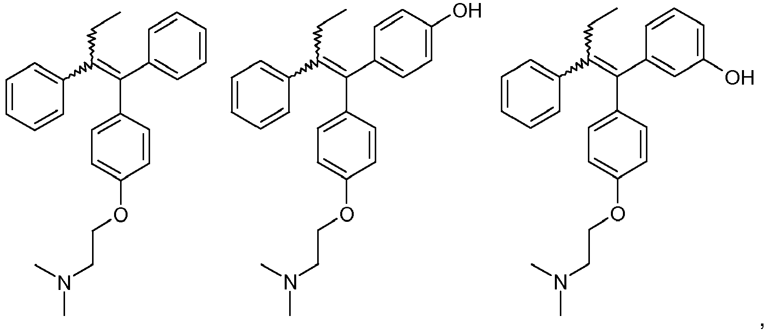 de préférence

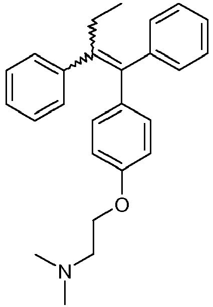

L'invention concerne un procédé de préparation d'un composé de formule (IV) comprenant la préparation d'un composé de formule (la) par mise en oeuvre du procédé de création de liaison C-C selon l'invention

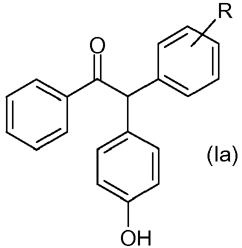

Dans un mode de réalisation, le procédé de préparation du composé de formule (IV) comprend les étapes suivantes :
a) préparation d'un composé de formule (la) par mise en oeuvre du procédé de création de liaison C-C selon l'invention ;
b) réaction du composé de formule (la) avec EtMgBr, notamment suivi d'une réaction d'élimination notamment avec de l'acide chlorhydrique puis isomérisation notamment avec le tert-butanoate de potassium dans le DMSO, pour obtenir le composé de formule (V) 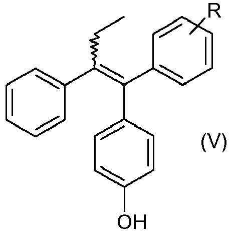
c) réaction du composé de formule (V) avec un composé de formule Y-(CH₂)₂N(Me)₂ dans laquelle Y représente un atome d'halogène, de préférence le chlore pour obtenir le composé de formule (IV).

Dans un autre mode de réalisation, le procédé de préparation du composé de formule (IV) comprend les étapes suivantes :
a) préparation d'un composé de formule (Ib) par mise en oeuvre du procédé de création de liaison C-C selon l'invention en présence d'un composé de formule Y-C₆H₄-O-(CH₂)₂N(Me)₂ dans laquelle Y représente un atome d'halogène, de préférence le chlore ; 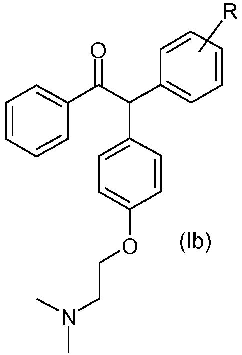
b) réaction du composé de formule (Ib) avec EtMgBr pour obtenir le composé de formule (IV), notamment suivi d'une réaction d'élimination notamment avec de l'acide chlorhydrique puis isomérisation notamment avec le tert-butanoate de potassium dans le DMSO.

La présente invention concerne également des composés de formule (la) et des composés de formule (Ib).

La présente invention va maintenant être décrite à l'aide d'exemples non limitatifs de mise en oeuvre du procédé selon l'invention.

Toutes les réactions ont été mises en oeuvre dans des tubes Sclenk de 35 ml ou dans des tubes Radley dans un Carousel « reaction stations RR98030 » sous azote. Le dioxane et le tert-butanol ont été distillés puis stockés en présence de tamis moléculaire 4Å sous azote. Les autres solvants sont distillés et stockés sous atmosphère d'azote. Les réactifs utilisés sont les suivants : deoxybenzoine 95% (Alfa Aesar), carbonate de cesium 99% (Alfa Aesar), le 2-acétylcyclohexanone, la phénantroline, le phosphate de potassium anhydre 97% (Alfa Aesar), Cul 99.999% (Aldrich).
Tous les autres matériaux solides ont été stockés en présence de P₄O₁₀ dans un dessicateur sous vide, à température ambiante et utilisés sans autre purification. Les composés de formule (II), notamment l'iodure d'aryle, sont des réactifs commerciaux. Les chromatographies sont réalisées avec du SDS 60 Å sur gel de silice (35-70 µm). Les chromatographies sur couche mince sont des gels de silice 60 F254.
Tous les produits sont caractérisés par RMN et GC/MS. Les spectres RMN sont réalisés à 20°C sur un spectromètre Bruker AC 400 MHz ou un spectromètre DRX-250 travaillant respectivement à 400 MHz pour ¹H, et 100 MHz pour ¹³C. Les déplacements chimiques sont reportés en ppm/TMS pour ¹H et ¹³C (δ 77.00 pour CDCl₃ signal). Les pics de premier ordre sont indiqués comme s (singulet), d (doublet), t (triplet), q (quadruplet). Les signaux complexes sont indiqués m (multiplet). Les analyses HRMS sont réalisés sur un spectromètre JEOL JMS-DX300 (3 keV, xenon) dans une matrice m-nitrobenzylalcool. Les points de fusion sont obtenus sur un appareil Büchi B-540.

### Exemple 1 : Mise en oeuvre du procédé selon l'invention

### Procédé (A)

Après plusieurs cycles standard d'évacuation et de purge des tubes avec un flux d'argon ou d'azote, le réacteur est chargé avec 0,01 mmol (19 mg) de Cul, 1,5 mmol d'un composé de formule (III), 2 mmol (650 mg) de Cs₂CO₃, 0,1 mmol de ligand et 1 mmol d'un composé de formule (II) si c'est un solide. Si le composé de formule (II) est un liquide, il est ajouté dans le milieu réactionnel à l'aide d'une seringue à température ambiante suivie de l'ajout de 1ml de tert-butanol anhydre ou dégazé ou de 1 ml de dioxane anhydre ou dégazé. Le réacteur est fermé sous pression positive d'azote ou d'argon. Le mélange réactionnel est agité et chauffé à 110°C pendant 24heures. Après refroidissement jusqu'à température ambiante, Le mélange réactionnel est acidifié avec une solution aqueuse d'HCl à 10% et extrait par deux fois avec de l'acétate d'éthyle. Les phases organiques obtenues sont réunies et lavées avec une solution de saumure, séchées sur Na₂SO₄, filtrées et concentrées sous vide. Le produit obtenu est purifié par chromatographie sur gel de silice.

### 1,2-diphenyl-2-p-tolylethanone (exemple 1.1):

Le procédé (A) est mis en oeuvre avec:
- composé de formule (II) : 4-iodotoluene (218 mg, 1.0 mmol),
- composé de formule (III) : deoxybenzoine,
- ligand : bathophenantroline,
- solvent : *t*-BuOH

Le rendement obtenu est de 85% (éluant chromatographie: éther de pétrole/diéthyléther = 95/5)

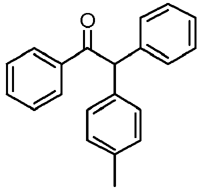

¹H RMN (400 MHz, CDCl₃) δ ppm 8,06-7,91 (m, 3H); 7,56- 7,43 (m, 2H); 7,44-7,34 (m, 2H); 7,34-7,20 (m, 3H); 7,19-7,09 (m, 4H); 6,00 (s, 1H); 2,30 (s, 3H).
¹³C RMN (100 MHz, CDCl₃) δ ppm 198,4; 139,4; 136,9; 136,8; 136,1; 133,0; 129,5; 129,1; 129,0; 129,0; 128,7; 128,6; 127,1; 59,1; 21,1.
HRMS calculée pour C₂₁H₁₉O (M+H) 287,1436. trouvée: 287,1427.

### 1,2-diphenyl-2-m-tolylethanone (exemple 1.2):

Le procédé (A) est mis en oeuvre avec:
- composé de formule (II) : 3-iodotoluene (130 µL, 1.0 mmol),
- composé de formule (III) : deoxybenzoine,
- ligand : bathophenantroline,
- solvent : *t*-BuOH

Le rendement obtenu est de 95% (éluant chromatographie: éther de pétrole/diéthyléther = 95/5).

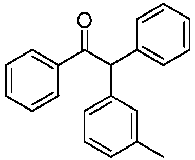

¹H RMN (400 MHz, CDCl3) δ ppm 7,96-7,87 (m, 3H), 7,63-7,53 (m, 2H), 7,47-7,38 (m, 2H), 7,36-7,28 (m, 1 H), 7,27-7,09 (m, 4H), 6,99 (dd, J = 13,7, 6,1 Hz, 2H), 5,93 (s, 1 H), 2,22 (s, 3H).
¹³C RMN (100 MHz, CDCl₃) δ ppm 198,3; 139,2; 138,9; 138,5; 136,9; 135,0; 133,0; 130,0; 129,8; 129,2; 129,1; 129,0; 128,7; 128,6; 128,0; 127,2; 126,2; 59,5; 21,6.
HRMS calculée pour C₂₁H₁₉O (M+H) 287,1436 trouvée: 287,1441.

### 2-(3,5-dimethylphenyl)-1,2-diphenylethanone (exemple 1.3):

Le procédé (A) est mis en oeuvre avec:
- composé de formule (II) : 3-iodoxylène (145 µL, 1.0 mmol),
- composé de formule (III) : deoxybenzoine,
- ligand : bathophenantroline,
- solvent : *t*-BuOH

Le rendement obtenu est de 95% (éluant chromatographie: éther de pétrole/diéthyléther = 95/5).

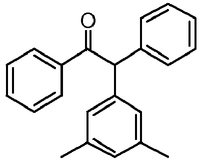

¹H RMN (400 MHz, CDCl₃) δ ppm 7,97-7,85 (m, 3H); 7,48-7,37 (m, 2H); 7,36-7,28 (m,2H); 7,27-7,11 (m, 3H); 6,89-6,70 (m, 3H); 5,89 (d, J = 2,55 Hz, 1 H); 2,18 (s, 6H).
¹³C RMN (100 MHz, CDCl₃) δ ppm 198,3; 139,2; 138,7; 138,2; 136,9; 132,9; 129,9; 129,1; 128,9; 128,6; 128,5; 127,0; 126,8; 59,3; 21,3.
HRMS calculée pour C₂₂H₂₁O (M+H) 301,1592 trouvée: 301,1589.

### 2-(4-methoxyphenyl)-1,2-diphenylethanone (exemple 1.4):

Le procédé (A) est mis en oeuvre avec:
- composé de formule (II) : 4-iodoanisole (234 mg, 1.0 mmol),
- composé de formule (III) : deoxybenzoine,
- ligand : phénantroline,
- solvent : *t*-BuOH

Le rendement obtenu est de 74% (éluant chromatographie: éther de pétrole/toluène = 1/1).

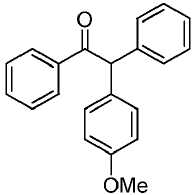

¹H RMN (400 MHz, CDCl₃) δ ppm 7,92 (dd, J = 8,4, 1,2 Hz, 2H), 7,48-7,39 (m, 1H), 7,37-7,29 (m, 2H), 7.24 (ddd, J = 7,1, 4,5, 1,2 Hz, 2H), 7,20-7,15 (m, 3H), 7,14-7,09 (m, 2H), 6,82-6,73 (m, 2H), 5,91 (s, 1 H), 3,69 (s, 3H).
¹³C RMN (100 MHz, CDCl₃) δ ppm 198,5; 158,7; 158,3; 139,5; 136,9; 133,0; 131,1; 130,2;129,1; 129,0; 128,7; 128,6; 127,1; 114,2; 58,7; 55,3.
HRMS calculée pour C₂₁H₁₉O₂ (M+H) 303,1385 trouvée: 303,1388.

### 2-(3-methoxyphenyl)-1,2-diphenylethanone (exemple 1.5):

Le procédé (A) est mis en oeuvre avec:
- composé de formule (II) : 3-iodoanisole (120µl, 1.0 mmol),
- composé de formule (III) : deoxybenzoine,
- ligand : bathophenantroline,
- solvent : *t*-BuOH

Le rendement obtenu est de 83% (éluant chromatographie: éther de pétrole/toluène = 1/1).

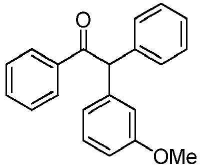

¹H RMN (400 MHz, CDCl₃) δ ppm 7,95-7,89 (m, 2H), 7,48-7,37 (m, 1H), 7,36-7,28 (m, 2H), 7,27-7,11 (m, 6H), 6,79 (dd, J = 7,4, 0,7 Hz, 1 H), 6,76-6,74 (m, 1 H), 6,71 (ddd, J = 8,2, 2,6, 0,7 Hz, 1 H), 5,93 (s, 1 H), 3,67 (s, 3H).
¹³C RMN (100 MHz, CDCl₃) δ ppm 195,7, 157,4, 138,1, 136,5, 134,5, 130,7, 127,3, 126,8, 126,6, 126,3, 126,3, 124,8, 119,2, 112,7, 110,0, 57,0, 52,8.
HRMS calculée pour C₂₁H₁₉O₂ (M+H) 303.1385 trouvée: 303.1375.

### 2-(2-methoxyphenyl)-1,2-diphenylethanone (exemple 1.6):

Le procédé (A) est mis en oeuvre avec:
- composé de formule (II) : 2-iodoanisole (130µl, 1.0 mmol),
- composé de formule (III) : deoxybenzoine,
- ligand : phenantroline,
- solvent : *t*-BuOH

Le rendement obtenu est de 50% (éluant chromatographie: éther de pétrole/toluène = 1/1).

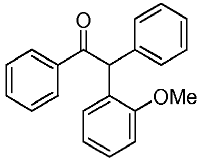

¹H RMN (400 MHz, CDCl₃) δ ppm 8,14-7,77 (m, 2H), 7,44-7,35 (m, 1H), 7,34-7,26 (m, 2H), 7,27-7,21 (m, 4H), 7,21-7,11 (m, 2H), 6,84 (dd, J = 7,4, 1,8 Hz, 1H), 6,78 (dd, J = 11,5, 4,3 Hz, 2H), 6,26 (s, 1H), 3,67 (s, 3H).
¹³C RMN (100 MHz, CDCl₃) δ ppm 198,9; 156,3; 137,6; 137,1; 132,7; 129,8; 128,8; 128,8; 128,5; 128,4; 127,2; 120,7; 110,4; 55,5; 53,1.
HRMS calculée pour C₂₁H₁₉O₂ (M+H) 303,1385 trouvée: 303,1378.

### 1-phenyl-2,2-dip-tolylethanone (exemple 1.7):

Le procédé (A) est mis en oeuvre avec:
- composé de formule (II) : 4-iodotoluène (218mg, 1,0 mmol),
- composé de formule (III) : 4-methylbenzylphenylcétone,
- ligand : bathophenantroline,
- solvent : *t*-BuOH

Le rendement obtenu est de 70% (éluant chromatographie: éther de pétrole/diéthyléther = 95/5).

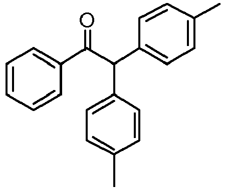

¹H RMN (400 MHz, CDCl₃) δ ppm 8,11-7,72 (m, 2H); 7,48-7,37 (m, 1H); 7,36-7,28 (m, 2H); 7,13-6,98 (m, 8H); 5,88 (s, 1 H); 2,23 (s, 6H).
¹³C RMN (100 MHz, CDCl₃) δ ppm 198,5; 137,0; 136,7; 136,3; 132,9; 129,4; 129,0; 128,6; 58,8; 21,1.
HRMS calculée pour C₂₂H₂₁O (M+H) 301,1592 Trouvée: 301,1588.

### 1-phenyl-2-m-tolyl-2-p-tolylethanone (exemple 1.8):

Le procédé (A) est mis en oeuvre avec:
- composé de formule (II) : 3-iodotoluène (130 µL, 1,0 mmol),
- composé de formule (III) : 4-methylbenzylphenylcétone,
- ligand : phenantroline,
- solvent : *t*-BuOH

Le rendement obtenu est de 54% (éluant chromatographie: éther de pétrole/diéthyléther = 95/5).

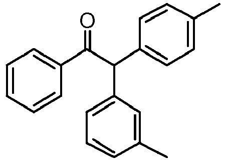

¹H RMN (400 MHz, CDCl₃) δ ppm 7,98-7,85 (m, 2H); 7,46-7,37 (m, 1H); 7,37-7,27 (m, 2H); 7,18-7,02 (m, 5H); 7,02-6,94 (m, 3H); 5,88 (s, 1H); 2,23 (s, 3H); 2,22 (s, 3H).
¹³C RMN (100 MHz, CDCl₃) δ ppm 198,4; 139,1; 138,3; 136,9; 136,7; 136,1; 132,9; 129,7; 129,4; 129,0; 128,9; 128,5; 127,9; 126,1; 59,0; 21,5; 21,0.
HRMS calculée pour C₂₂H₂₁O (M+H) 301,1592 Trouvée: 301,1602.

### 2-(3,5-dimethylphenyl)-1-phenyl-2-p-tolylethanone (exemple 1.9):

Le procédé (A) est mis en oeuvre avec:
- composé de formule (II) : 4-iodoxylène (145 µL, 1,0 mmol),
- composé de formule (III) : 4-methylbenzylphenylcétone,
- ligand : bathophenantroline,
- solvent : *t*-BuOH

Le rendement obtenu est de 90% (éluant chromatographie: éther de pétrole/diéthyléther = 95/5).

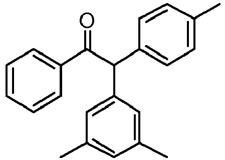

¹H RMN (250 MHz, CDCl₃) δ ppm 8,01-7,84 (m, 2H), 7,50-7,38 (m, 1H), 7,39-7,27 (m, 2H), 7,13-7,00 (m, 4H), 6,81 (app s, 3H), 5,86 (s, 1H), 2,24 (s, 3H), 2,19 (s, 6H).
¹³C RMN (100 MHz, CDCl₃) δ ppm 198,8, 139,0, 138,4, 137,0, 136,6, 136,2, 132,8, 129,4, 129,1, 128,9, 128,6, 126,9, 58,9, 30,8, 21,1.
HRMS calculée pour C₂₃H₂₃O₂ (M+H) 315,1749 Trouvée: 315,1744.

### 2-(4-methoxyphenyl)-1-phenyl-2-p-tolylethanone (exemple 1.10):

Le procédé (A) est mis en oeuvre avec:
- composé de formule (II) : 4-iodoanisole (234mg, 1,0 mmol),
- composé de formule (III) : 4-methylbenzylphenylcétone,
- ligand : bathophenantroline,
- solvent : *t*-BuOH

Le rendement obtenu est de 66% (éluant chromatographie: éther de pétrole/toluène=1/1).

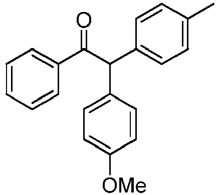

¹H RMN (400 MHz, CDCl₃) δ ppm 8,08-7,74 (m, 2H), 7,47-7,36 (m, 1H), 7,36-7,25 (m, 2H), 7,18-6,97 (m, 6H), 6,81-6,70 (m, 2H), 5,87 (s, 1H), 3,67 (s, 3H), 2,22 (s, 3H).
¹³C RMN (100 MHz, CDCl₃) δ ppm 198,7; 158,6; 136,9; 136,7; 136,5; 133,0; 131,4; 130,2; 129,5; 129,0; 129,0; 128,6; 114,1; 58,3; 55,2; 31,0; 21,1.
HRMS calculée pour C₂₂H₂₁O₂ (M+H) 317,1542, Trouvée: 317,1531.

### 1-(4-chlorophenyl)-2-phenyl-2-p-tolylethanone (exemple 1.11):

Le procédé (A) est mis en oeuvre avec:
- composé de formule (II) : 4-iodotoluène (218mg, 1,0 mmol),
- composé de formule (III) : 4-chlorophenylbenzylcétone,
- ligand : phenantroline,
- solvent : *dioxane*

Le rendement obtenu est de 95% (éluant chromatographie: éther de pétrole/diéthyléther = 95/5).

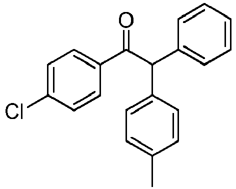

¹H RMN (400 MHz, CDCl₃) δ ppm 7,94-7,73 (m, 2H), 7,31-7,24 (m, 2H), 7,25-7,19 (m, 2H), 7,19-7,12 (m, 3H), 7,09-7,01 (m, 4H), 5,84 (s, 1H), 2,22 (s, 3H).
¹³C RMN (100 MHz, CDCl₃) δ ppm 197,2; 139,4; 139,0; 137,0; 135,7; 135,2; 130,4; 129,6; 129,1; 129,0; 128,9; 128,8; 127,2; 59,3; 21,1.
HRMS calculée pour C₂₁H₁₈OCl (M+H) 321,1046 Trouvée: 321,1023.

### 1-(4-chlorophenyl)-2-phenyl-2-m-tolylethanone (exemple 1.12):

Le procédé (A) est mis en oeuvre avec:
- composé de formule (II) : 3-iodotoluène (130 µL, 1,0 mmol),
- composé de formule (III) : 4-chlorophenylbenzylcétone,
- ligand : phenantroline,
- solvent : *t*-BuOH

Le rendement obtenu est de 96% (éluant chromatographie: éther de pétrole/diéthyléther = 95/5).

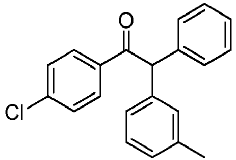

¹H RMN (400 MHz, CDCl₃) δ ppm 7,98-7,69 (m, 2H), 7,33-7,27 (m, 2H), 7,27-7,21 (m, 2H), 7,21-7,10 (m, 4H), 7,03-6,93 (m, 3H), 5,85 (s, 1H), 2,23 (s, 3H)
¹³C RMN (100 MHz, CDCl₃) δ ppm, 197,0, 139,5, 138,9, 138,6, 138,5, 135,2, 130,4, 129,7, 129,1, 128,9, 128,7, 128,7, 128,1, 127,2, 126,1, 59,6, 21,5.
HRMS calculée pour C₂₁H₁₈OCl (M+H) 321,1046 Trouvée: 321,1046.

### 1-(4-chlorophenvl)-2-(3,5-dimethylphenyl)-2-phenylethanone (exemple 1.13):

Le procédé (A) est mis en oeuvre avec:
- composé de formule (II) : 3-iodoxylène (145 µL, 1,0 mmol),
- composé de formule (III) : 4-chlorophenylbenzylcétone,
- ligand : phenantroline,
- solvent : dioxane

Le rendement obtenu est de 95% (éluant chromatographie: éther de pétrole/diéthyléther = 95/5).

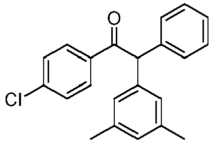

¹H RMN (400 MHz, CDCl₃) δ ppm 8,04-7,67 (m, 3H), 7,47-7,37 (m, 1H), 7,32-7,25 (m,2H),7,25-7,20 (m, 1H), 7,20-7,12 (m, 2H), 6,85-6,75 (m, 3H), 5,81 (s, 1H), 2,18 (s, 6H).
¹³ CRMN (100 MHz, CDCl₃) δ ppm 197,2, 139,5, 139,0, 138,4, 135,2, 135,1, 131,3, 130,4, 129,2, 129,1, 128,9, 128,7, 126,8, 59,4, 21,5.
HRMS calculée pour C₂₂H₂₀OCl (M+H) 335,1203 Trouvée: 335,1189.

### 1-(4-chlorophenyl)-2-(4-methoxyphenyl)-2-phenylethanone (exemple 1.14):

Le procédé (A) est mis en oeuvre avec:
- composé de formule (II) : 4-iodoanisole (234mg, 1,0 mmol),
- composé de formule (III) : 4-chlorophenylbenzylcétone,
- ligand : phenantroline,
- solvent : dioxane

Le rendement obtenu est de 70% (éluant chromatographie: éther de pétrole/toluène = 1/1).

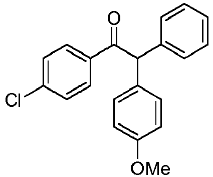

¹H RMN (400 MHz, CDCl₃) δ ppm 8,00-7,68 (m, 2H); 7,32-7,27 (m, 2H); 7,26-7,21 (m, 2H); 7,20-7,12 (m, 3H); 7,12-7,06 (m, 2H); 6,85-6,70 (m, 2H); 5,83 (s, 1H); 3,69 (s, 3H).
¹³C RMN (100 MHz, CDCl₃) δ ppm 197,2; 158,8; 139,4; 139,1; 135,1; 130,7; 130,3; 130,1; 129,0; 128,9; 128,7; 127,2; 114,2; 58,7; 55,2.
HRMS calculée pour C₂₁H₁₈O₂Cl (M+H) 337,0995 Trouvée: 337,0988.

### 1-(4-chlorophenyl)-2-(3-methoxyphenyl)-2-phenylethanone (exemple 1.15):

Le procédé (A) est mis en oeuvre avec:
- composé de formule (II) : 3-iodoanisole (120 µL, 1,0 mmol),
- composé de formule (III) : 4-chlorophenylbenzylcétone,
- ligand : phenantroline,
- solvent : dioxane

Le rendement obtenu est de 75% (éluant chromatographie: éther de pétrole/toluène = 95/5).

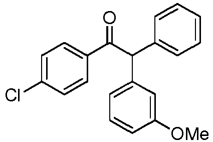

¹H RMN (400 MHz, CDCl₃) δ ppm 7,88-7,80 (m, 2H), 7,34-7,26 (m, 2H), 7,26-7,21 (m, 2H), 7,20-7,14 ( m, 4H), 6,82-6,74 (m, 1 H), 6,74-6,69 (m, 2H), 5,84 (s, 1 H), 3,67 (s, 3H). ¹³C RMN (100 MHz, CDCl₃) δ ppm 196,8, 160,0, 140,2, 139,5, 138,6, 135,1, 130,4, 129,8, 129,1, 129,0, 128,8, 127,3, 121,6, 115,2, 112,4, 59,5, 55,2.
HRMS calculée pour C₂₁H₁₈O₂Cl (M+H) 337,0995 Trouvée: 337,0999.

### 1-(4-chlorophenyl)-2-(2-methoxyphenyl)-2-phenylethanone (exemple 1.16):

Le procédé (A) est mis en oeuvre avec:
- composé de formule (II) : 2-iodoanisole (130 µL, 1,0 mmol),
- composé de formule (III) : 4-chlorophenylbenzylcétone,
- ligand : phenantroline,
- solvent : dioxane

Le rendement obtenu est de 56% (éluant chromatographie: éther de pétrole/toluène = 1/1).

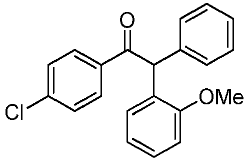

¹H RMN (400 MHz, CDCl₃) δ ppm 7,89-7,83 (m, 2H), 7,33-7,24 (m, 4H), 7,24-7,19 (m, 3H), 7,18-7,14 (m, 1H), 6,85-6,75 (m, 3H), 6,18 (s, 1H), 3,69 (s, 3H).
¹³C RMN (100 MHz, CDCl₃) δ ppm 197,6, 156,2, 139,0, 137,3, 135,4, 131,0, 130,2, 129,7, 128,8, 128,8, 128,5, 128,4, 127,4, 120,7, 110,5, 55,5, 53,2.
HRMS calculée pour C₂₁H₁₈O₂Cl (M+H) 337,0995 Trouvée: 321,0991.

### 2-(3,5-dimethylphenyl)-2-(2-fluorophenyl)-1-phenylethanone (exemple 1.17):

Le procédé (A) est mis en oeuvre avec:
- composé de formule (II) : 3-iodoxylène (145 µL, 1,0 mmol),
- composé de formule (III) : 2-fluorobenzylphenylcétone,
- ligand : bathophenantroline,
- solvent : *t*-BuOH

Le rendement obtenu est de 96% (éluant chromatographie: éther de pétrole/diéthyléther = 95/5).

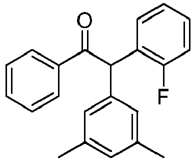

¹H RMN (250 MHz, CDCl₃) δ ppm 8,04-7,82 (m, 2H), 7,46-7,35 (m, 1H), 7,34-7,27 (m, 2H), 7,18-7,09 (m, 1H), 7,04-6,90 (m, 3H), 6,87-6,80 (m, 3H), 6,16 (s, 1H), 2,19 (s, 6H). ¹³C RMN (100 MHz, CDCl₃) δ ppm 197,6, 161,5, 159,0, 138,6, 136,8, 136,5, 133,1, 130,9, 130,8, 129,3, 129,0, 129,0, 129,0, 128,6, 127,2, 127,1, 124,2, 124,1, 115,3, 115,0, 52,0, 21,4.

### 2-(3,5-dimethylphenyl)-2-(4-chlorophenyl)-1-phenylethanone (exemple 1.18):

Le procédé (A) est mis en oeuvre avec:
- composé de formule (II) : 3-iodoxylène (145 µL, 1,0 mmol),
- composé de formule (III) : 4-chlorobenzylphenylcétone,
- ligand : bathophenantroline,
- solvent : *t*-BuOH

Le rendement obtenu est de 99% (éluant chromatographie: éther de pétrole/diéthyléther = 95/5).

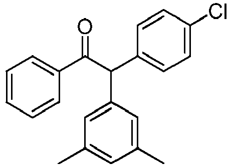

¹H RMN (250 MHz, CDCl₃) δ ppm 8,04-7,78 (m, 2H), 7,52-7,39 (m, 1H), 7,39-7,31 (m, 1 H), 7,25-7,18 (m, 2H), 7,15-7,09 (m, 2H), 6,82 (br s, 1H), 6,80 (br s, 2H), 5,85 (s, 1H), 2,20 (s, 6H).
¹³C RMN (100 MHz, CDCl₃) δ ppm 198,0, 138,5, 138,3, 137,9, 136,7, 133,2, 133,0, 130,6, 129,2, 129,0, 128,7, 128,7, 126,7, 58,7, 21,3.

### Procédé B

Après plusieurs cycles standard d'évacuation et de purge des tubes avec un flux d'argon ou d'azote, le réacteur est chargé avec 0,01 mmol (19 mg) de Cul, 1,5 mmol d'un composé de formule (III), 2 mmol (650 mg) de Cs₂CO₃, 0,1 mmol de 2-acétylcyclohexanone et 1 mmol d'un composé de formule (II) si c'est un solide. Si le composé de formule (II) est un liquide il est ajouté dans le milieu réactionnel à l'aide d'une seringue à température ambiante avec du 2-acétylcyclohexanone (0,1 mmol, 13 µL) suivie de l'ajout de 1ml de tert-butanol anhydre et dégazé. Le réacteur est fermé sous pression positive d'azote ou d'argon. Le mélange réactionnel est agité et chauffé à 70 ou 90°C pendant 24heures. Après refroidissement jusqu'à température ambiante, 13 µL de 1,3-dimethoxybenzène (standard interne) puis 1 ml d'acétate d'éthyle sont ajoutés. Un échantillon du milieu réactionnel est prélevé et acidifié avec une solution aqueuse HCl à 10%, la phase organique est ensuite filtrée sur célite et le résidu est lavé à l'acétate d'éthyle. Le filtrat est analysé par RMN du proton. Le mélange réactionnel est acidifié avec une solution aqueuse d'HCl à 10% et extrait par deux fois avec de l'acétate d'éthyle. Les phases organiques obtenues sont réunies et lavées avec une solution de saumure, séchées sur Na₂SO₄, filtrées et concentrées sous vide. Le produit obtenu est purifié par chromatographie sur gel de silice.

### 1,2-diphenyl-2-(4-fluorophenyl)ethanone (exemple 1.19) :

Le procédé (B) a été suivi à 70°C avec:
Composé de formule (II) : 4-iodofluorobenzene (115 µL, 1,0 mmol)
Composé de formule (III) : deoxybenzoin

Le produit (I) est obtenu avec un rendement de 75% (éluant: éther de pétrole/diéthyléther = 95/5).

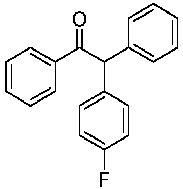

¹H RMN (400 MHz, CDCl₃) δ ppm 7,90 (td, J = 8,6, 1,6 Hz, 2H), 7,46-7,35 (m, 1H), 7,36-7,27 (m, 2H), 7,27-7,19 (m, 2H), 7,19-7,10 (m, 5H), 6,98-6,83 (m, 2H), 5,93 (s, 1H)
¹³C RMN (100 MHz, CDCl₃) δ ppm 198,0; 163,0; 160,6; 138,8; 136,5; 134,9; 134,8; 133,1; 130,7; 130,6; 128,9; 128,9; 128,8; 128,6; 127,2; 115,6; 115,4; 58,5.
HRMS calculée par C₂₀H₁₆OF (M+H) 291,1185 Trouvée: 291,1173.

### 1,2-diphenyl-2-(4-bromophenyl)ethanone (exemple 1.20) :

Le procédé (B) a été suivi à 70°C avec:
Composé de formule (II) : 4-iodobromobenzene (283 mg, 1,0 mmol)
Composé de formule (III) : deoxybenzoin

Le produit (I) est obtenu avec un rendement de 60% (éluant: éther de pétrole/diéthyléther = 95/5).

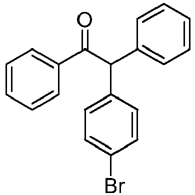

¹H RMN (400 MHz, CDCl₃) δ ppm 7,90 (td, J = 8,6, 1,7 Hz, 2H), 7,49-7,40 (m, 1H), 7,39-7,30 (m, 4H), 7,29-7,21 (m, 2H), 7,21-7,15 (m, 3H), 7,09-7,03 (m, 2H), 5,91 (s, 1H).
¹³C RMN (100 MHz, CDCl₃) δ ppm 197,8, 138,5, 138,2, 136,5, 133,3, 131,8, 130,9, 129,0, 129,0, 129,0, 127,4, 121,3, 58,8.

### 1,2-diphenyl-2-(4-(trifluoromethyl)phenyl)ethanone (exemple 1.21) :

Le procédé (B) a été suivi à 70°C avec:
Composé de formule (II) : 4-iodobenzotrifluoride (150 µL, 1,0 mmol)
Composé de formule (III) : deoxybenzoin

Le produit (I) est obtenu avec un rendement de 55% (éluant: éther de pétrole/diéthyléther = 95/5).

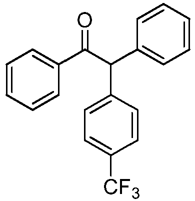

¹H RMN (400 MHz, CDCl₃) δ ppm 7,94-7,86 (m, 2H), 7,47 (d, J = 8,1 Hz, 2H), 7,45-7,39 (m, 1H), 7,35-7,25 (m, 1H), 7,24 (ddd, J = 7,1, 4,4, 1,7 Hz, 2H), 7,21-7,16 (m, 3H), 6,00 (s, 1 H)
¹³C RMN (100 MHz, CDCl₃) δ ppm 197,5, 143,4, 138,2, 136,6, 133,4, 129,6, 129,1, 129,1, 129,0, 128,8, 127,6, 125,6, 59,2.

### 1,2-diphenyl-2-(3-fluorophenyl)ethanone (exemple 1.22) :

Le procédé (B) a été suivi à 70°C avec:
Composé de formule (II) : 3-iodofluorobenzene (120 µL, 1,0 mmol)
Composé de formule (III) : deoxybenzoin

Le produit (I) est obtenu avec un rendement de 48% (éluant: éther de pétrole/diéthyléther = 95/5).

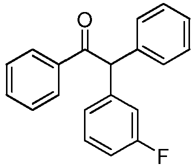

¹H RMN (400 MHz, CDCl₃) δ ppm 8,03-7,77 (m, 2H), 7,50-7,30 (m, 3H), 7,29-7,11 (m, 6H), 7,03-6,77 (m, 3H), 5,94 (s, 1 H)
¹³C RMN (100 MHz, CDCl₃) δ ppm 197,6, 164,9, 160,9, 141,7, 141,6, 138,4, 136,6, 133,3, 130,1, 130,0, 129,1, 129,0, 128,7, 127,5, 124,9, 124,8, 116,5, 116,1, 114,3, 114,0, 59,0.

### 1,2-diphenyl-2-(3-(trifluoromethyl)phenyl)ethanone (exemple 1.23) :

Le procédé (B) a été suivi à 70°C avec:
Composé de formule (II) : 3-iodobenzotrifluoride (150 µL, 1,0 mmol)
Composé de formule (III) : deoxybenzoin

Le produit (I) est obtenu avec un rendement de 60% (éluant: éther de pétrole/diéthyléther = 95/5).

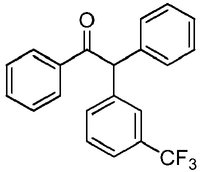

¹H RMN (400 MHz, CDCl₃) δ ppm 7,92 (td, J = 8,6, 1,7 Hz, 2H), 7,49-7,41 (m, 3H), 7,40-7,30 (m, 4H), 7,27 (ddd, J = 7,1, 4,4, 1,7 Hz, 2H), 7,23-7,18 (m, 3H), 6,01 (s, 1H)
¹³C RMN (100 MHz, CDCl₃) δ ppm 197,5, 140,2, 138,2, 136,4, 133,4, 132,7, 129,1, 129,0, 129,0, 128,8, 127,6, 125,9, 124,1, 59,1.

### 2-(4-fluorophenyl)-1-phenyl-2-p-tolylethanone (exemple 1.24) :

Le procédé (B) a été suivi à 90°C avec:
Composé de formule (II) : 4-fluoroiodobenzene (115 µL, 1,0 mmol)
Composé de formule (III) : 4-methylbenzylphenylcetone

Le produit (I) est obtenu avec un rendement de 57% (éluant: éther de pétrole/diéthyléther = 95/5).

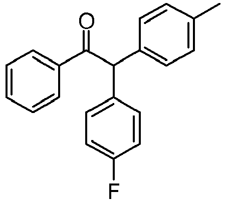

¹H RMN (400 MHz, CDCl₃) δ ppm 7,91 (td, J = 8,55, 1,69, 1,69 Hz, 2H), 7,48-7,41 (m, 1H), 7,37-7,30 (m, 2H), 7,20-7,10 (m, 3H), 7,10-7,04 (m, 3H), 6,96-6,88 (m, 2H), 5,90 (s, 1 H), 2,24 (s, 3H).
¹³C RMN (100 MHz, CDCl₃) δ ppm 198,2, 163,1, 160,7, 137,1, 136,7, 135,9, 135,2, 133,2, 130,7, 130,7, 129,7, 129,0, 128,8, 128,7, 115,6, 115,4, 58,3, 21,1.
HRMS calculée par C₂₁H₁₈OF (M+H) 305,1342. Trouvée: 305,1335.

### 2-(3-fluorophenyl)-1-phenyl-2-p-tolylethanone (exemple 1.25) :

Le procédé (B) a été suivi à 90°C avec:
Composé de formule (II) : 3-fluoroiodobenzene (115 µL, 1,0 mmol)
Composé de formule (III) : 4-methylbenzylphenylcetone

Le produit (I) est obtenu avec un rendement de 54% (éluant: éther de pétrole/diéthyléther = 95/5).

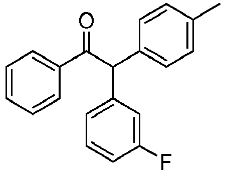

¹H RMN (400 MHz, CDCl₃) δ ppm 7,90 (td, J = 8,6, 1,7 Hz, 2H), 7,48-7,37 (m, 1 H), 7,36-7,27 (m, 2H), 7,22-7,13 (m, 1H), 7,12-7,02 (m, 4H), 6,98-6,92 (m, 1H), 6,91-6,80 (m, 2H), 5,90 (s, 1H), 2,22 (s, 3H)
¹³C RMN (100 MHz, CDCl₃) δ ppm 197,8, 164,1, 161,7, 141,9, 137,2, 136,7, 135,4, 133,2, 130,0, 129,9, 129,7, 129,0, 128,9, 128,7, 124,8, 124,8, 116,4, 116,2, 114,2, 113,9, 58,6, 21,2.

### 1-phenyl-2-p-tolyl-2-(3-(trifluoromethyl)phenyl)ethanone (exemple 1.26) :

Le procédé (B) a été suivi à 90°C avec:
Composé de formule (II) : 3-iodobenzotrifluoride (150 µL, 1,0 mmol)
Composé de formule (III) : 4-methylbenzylphenylcetone

Le produit (I) est obtenu avec un rendement de 40% (éluant: éther de pétrole/diéthyléther = 95/5).

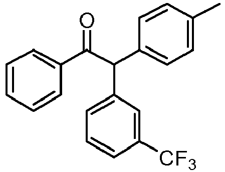

¹H RMN (400 MHz, CDCl₃) δ ppm 7,92 (td, J = 8,6, 1,6 Hz, 2H), 7,52-7,40 (m, 3H), 7,40-7,30 (m, 4H), 7,14-7,04 (m, 4H), 5,97 (s, 1H), 2,24 (s, 3H).
¹³C RMN (100 MHz, CDCl₃) δ ppm 197,6, 140,4, 137,4, 136,5, 135,1, 133,3, 132,7, 129,9, 129,0, 129,0, 128,8, 128,7, 125,9, 124,0, 58,7, 21,1.
HRMS calculée par C₂₂H₁₈OF₃ (M+H) 355,1310 Trouvée: 355,1308.

### 1-(4-chlorophenyl)-2-(4-fluorophenyl)-2-phenylethanone (exemple 1.27) :

Le procédé (B) a été suivi à 70°C avec:
Composé de formule (II) : 4-fluoroiodobenzene (115 µL, 1,0 mmol)
Composé de formule (III) : 4-chlorophenylbenzylcetone

Le produit (I) est obtenu avec un rendement de 90% (éluant: éther de pétrole/diéthyléther = 95/5).

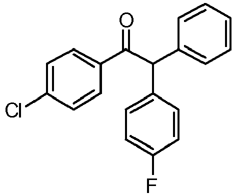

¹H RMN (400 MHz, CDCl₃) δ ppm 7,91-7,74 (m, 2H), 7,31-7,24 (m, 2H), 7,24-7,20 (m, 2H), 7,19-7,07 (m, 5H), 6,96-6,85 (m, 2H), 5,85 (s, 1H)
¹³C RMN (100 MHz, CDCl₃) δ ppm 196,9, 163,3, 160,9, 139,7, 138,6, 134,9, 134,6, 130,8, 130,7, 130,4, 129,0, 129,0, 127,5, 115,8, 115,5, 58,7.
HRMS calculée par C₂₀H₁₅OFCl (M+H) 325,0795 Trouvée: 325,0791.

### 1,2-bis(4-chlorophenyl)-2-phenylethanone (exemple 1.28) :

Le procédé (B) a été suivi à 70°C avec:
Composé de formule (II) : 4-chloroiodobenzene (239 mg, 1,0 mmol)
Composé de formule (III) : 4-chlorophenylbenzylcetone

Le produit (I) est obtenu avec un rendement de 75% (éluant: éther de pétrole/diéthyléther = 95/5).

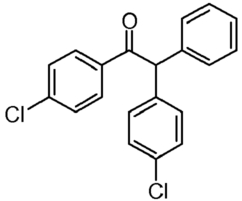

¹H RMN (400 MHz, CDCl₃) δ ppm 7,97-7,86 (m, 2H), 7,50-7,41 (m, 1H), 7,38-7,30 (m, 2H), 7,29-7,21 (m, 3H), 7,21-7,16 (m, 3H), 7,15-7,09 (m, 2H), 5,93 (s, 1H).
¹³C RMN (100 MHz, CDCl₃) δ ppm 197,8, 138,6, 137,7, 136,5, 133,3, 133,2, 130,6, 129,0, 129,0, 129,0, 128,8, 128,7, 127,4, 58,7.
HRMS calculée par C₂₀H₁₄OCl₂ (M+H) 341,0005. Trouvée: 341,0011.

### 1-(4-chlorophenyl)-2-phenyl-2-(4-(trifluoromethyl)phenyl)ethanone (exemple 1.29) :

Le procédé (B) a été suivi à 70°C avec:
Composé de formule (II) : 4-iodobenzotrifluoride (150 µL, 1,0 mmol)
Composé de formule (III) : 4-chlorophenylbenzylcetone

Le produit (I) est obtenu avec un rendement de 35% (éluant: éther de pétrole/diéthyléther = 95/5).

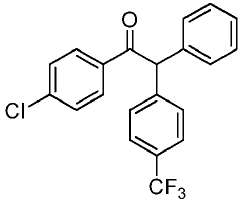

¹H RMN (400 MHz, CDCl₃) δ ppm 7,86-7,78 (m, 2H), 7,47 (d, J = 8,1 Hz, 2H), 7,31-7,21 (m, 6H), 7,21-7,14 (m, 3H), 5,92 (s, 1 H).
¹³C RMN (100 MHz, CDCl₃) δ ppm 196,3, 142,9, 139,9, 137,8, 134,7, 130,5, 129,6, 129,3, 129,1, 129,0, 127,8, 125,6, 59,3.
HRMS calculée par C₂₁H₁₅OF₃Cl (M+H) 375,0764. Trouvée: 375,0770.

### 1-(4-chlorophenyl)-2-(3-fluorophenyl)-2-phenylethanone (exemple 1.30) :

Le procédé (B) a été suivi à 70°C avec:
Composé de formule (II) : 3-fluoroiodobenzene (120 µL, 1,0 mmol)
Composé de formule (III) : 4-chlorophenylbenzylcetone

Le produit (I) est obtenu avec un rendement de 55% (éluant: éther de pétrole/diéthyléther = 95/5).

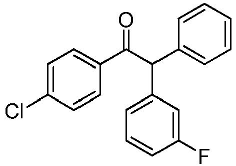

¹H RMN (400 MHz, CDCl₃) δ ppm 7,91-7,79 (m, 2H), 7,35-7,24 (m, 4H), 7,23-7,15 (m, 4H), 6,95 (td, J = 7,6, 1,4 Hz, 1 H), 6,92-6,84 (m, 2H), 5,88 (s, 1 H).
¹³C RMN (100 MHz, CDCl₃) δ ppm 196,4, 164,2, 161,7, 141,2, 139,8, 138,0, 134,8, 130,4, 130,2, 130,1, 129,2, 129,1, 129,0, 127,6, 124,8, 116,3, 114,3, 59,1.
HRMS calculée par C₂₀H₁₅OClF (M+H) 325,0795 Trouvée: 325,0803.

### 1-(4-chlorophenyl)-2-phenyl-2-(3-(trifluoromethyl)phenyl)ethanone (exemple 1.31) :

Le procédé (B) a été suivi à 70°C avec:
Composé de formule (II) : 3-iodobenzotrifluoride (150 µL, 1,0 mmol)
Composé de formule (III) : 4-chlorophenylbenzylcetone

Le produit (I) est obtenu avec un rendement de 20% (éluant: éther de pétrole/diéthyléther = 95/5).

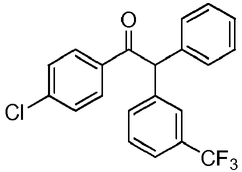

¹H RMN (400 MHz, CDCl₃) δ ppm 7,86-7,79 (m, 2H), 7,47-7,39 (m, 2H), 7,36-7,31 (m, 2H), 7,30-7,26 (m, 2H), 7,26-7,22 (m, 2H), 7,21-7,15 (m, 3H), 5,93 (s, 1H)
¹³C RMN (100 MHz, CDCl₃) δ ppm 196,3, 139,9, 139,9, 137,9, 134,7, 132,7, 130,4, 129,3, 129,1, 128,9, 127,8, 125,9, 124,2, 59,2.
HRMS calculée par C₂₁H₁₅OF₃Cl (M+H) 375,0764. Trouvée: 375,0766.

### 2-(4-fluorophenyl)-2-(4-chlorophenyl)-1-phenylethanone (exemple 1.32) :

Le procédé (B) a été suivi à 70°C avec:
Composé de formule (II) : 4-fluoroiodobenzene (115 µL, 1,0 mmol)
Composé de formule (III) : 4-chlorophenylbenzylcetone

Le produit (I) est obtenu avec un rendement de 95% (éluant: éther de pétrole/diéthyléther = 95/5).

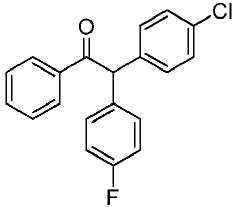

¹H RMN (400 MHz, CDCl₃) δ ppm 7,97-7,83 (m, 2H), 7,54-7,41 (m, 1H), 7,40-7,29 (m, 2H), 7,28-7,05 (m, 6H), 7,01-6,87 (m, 2H), 5,91 (s, 1H).
¹³C RMN (100 MHz, CDCl₃) δ ppm 197,7, 163,3, 160,8, 137,4, 136,3, 134,4, 133,4, 133,4, 130,7, 130,6, 130,4, 129,0, 129,0, 128,8, 115,9, 115,7, 57,7.

### Procédé C

Après plusieurs cycles standard d'évacuation et de purge des tubes avec un flux d'argon ou d'azote, le réacteur est chargé avec 0,01 mmol (19 mg) de Cul, 1,5 mmol d'un composé de formule (III), 2 mmol (650 mg) de Cs₂CO₃, 0,1 mmol de bathophenanthroline et 1 mmol d'un composé de formule (II) si c'est un solide. Si le composé de formule (II) est un liquide il est ajouté dans le milieu réactionnel à l'aide d'une seringue sous flux d'azote à température ambiante suivie de l'ajout de 1ml de tert-butanol anhydre et dégazé. Le réacteur est fermé sous pression positive d'azote ou d'argon. Le mélange réactionnel est agité et chauffé à 110°C pendant 24 heures. Après refroidissement jusqu'à température ambiante, 13 µL de 1,3-dimethoxybenzène (standard interne) puis 1 ml d'acétate d'éthyle sont ajoutés. Un échantillon du milieu réactionnel est prélevé et acidifié avec une solution aqueuse HCl à 10%, la phase organique est ensuite filtrée sur célite et le résidu est lavé à l'acétate d'éthyle. Les phases organiques obtenues sont réunies et lavées avec une solution de saumure, séchées sur Na₂SO₄, filtrées et concentrées sous vide. Le produit obtenu est purifié par chromatographie sur gel de silice et analysé par RMN.

**Les résultats obtenus sont résumés dans le tableau ci-dessous :**

| Exemples | Composé de formule (I) | Composé de formule (II) | Composé de formule (III) | Rendement (%) |
|---|---|---|---|---|
| Exemple 1.33 | 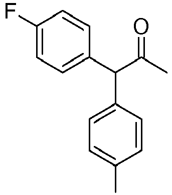 | 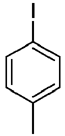 | 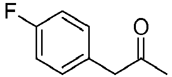 | 10 |
| Exemple 1.34 | 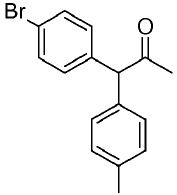 | 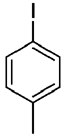 | 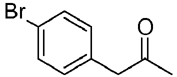 | 17 |
| Exemple 1.35 | 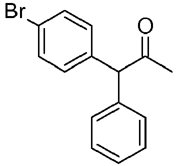 | 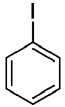 | 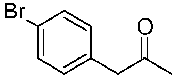 | 15 |
| Exemple 1.36 | 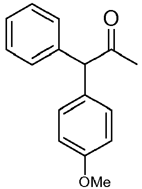 | 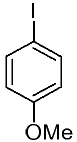 | 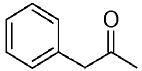 | 11 |
| Exemple 1.37 | 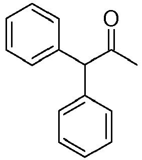 | 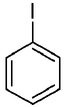 | 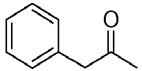 | 11 |

### Exemple 2 : Influence du catalyseur, du solvant et de la base

Le procédé selon l'invention a été mis en oeuvre selon la procédure A avec différents solvants, différentes bases (2 mmol) et différents catalyseurs (10% en mole par rapport au composé de formule (III) avec pour composé de formule (II) le 4-méthyliodobenzène (1 mmol), pour composé de formule (III) la deoxybenzoine (1,5 mmol) et pour ligand le composé L1 (0,1 mmol). Le rendement est déterminé par RMN avec le 1,3 dimethoxybenzène en tant que standard interne.

Les résultats sont précisés dans le tableau (1) :

| solvant | Catalyseur | Base | Rendement [%] |
|---|---|---|---|
| DMF ou CH₃CN | Cul | Cs₂CO₃ | 10 |
| DMA | Cul | Cs₂CO₃ | 17 |
| *t*-BuOH | Cul | Cs₂CO₃ | 52 |
| dioxane | Cul | Cs₂CO₃ | 70 |
| dioxane | Cul | Rb₂CO₃ ou K₂CO₃ | 18-21 |
| dioxane | Cu(acac)₂ | Cs₂CO₃ | 30 |
| dioxane | Cu(OTf)₂ ou Cu₂O | Cs₂CO₃ | 43-45 |
| dioxane | CuCl, Cu(Oac)₂, CuBr₂, ou CuCN | Cs₂CO₃ | 48-51 |

| | | | |
|---|---|---|---|
| Tableau 1 | | | |

Ces résultats montrent que de façon avantageuse le procédé de l'invention peut être mis en oeuvre avec divers catalyseurs, bases et solvants.

### Exemple 3 : Influence du ligand et du solvant

Le procédé selon l'invention a été mis en oeuvre selon la procédure A à 110°C avec différents solvants et différents ligands (10 % en mole par rapport au composé de formule (III), avec pour composé de formule (II) le 4-méthyliodobenzène (1 mmol), pour composé de formule (III) la deoxybenzoine (1,5 mmol) pour base le Cs₂CO₃ (2 mmol) et pour catalyseur Cul (10% en mole par rapport au composé de formule (III). Le rendement est déterminé par RMN avec le 1,3 dimethoxybenzène en tant que standard interne.

Les résultats sont précisés dans le tableau (2) :

**Tableau 2**

| Ligand | rendement [%] | | Ligand | rendement [%] | |
|---|---|---|---|---|---|
| | Solvant dioxane | Solvant t-BuOH | | Solvant dioxane | Solvant t-BuOH |
| - | 8 | 6 | L5 | 29 | 5 |
| L1 | 70 | 52 | L6 | 23 | 8 |
| L2 | 27 | 46 | L7 | 20 | 17 |
| L3 | 3 | 47 | L8 | 24 | 58 |
| L4 | 68 | 84 | L9 | 11 | 8 |

Ces résultats montrent que de façon avantageuse le procédé de l'invention peut être mis en oeuvre avec divers ligands et solvants et met en évidence quelques combinaisons préférées.

### Exemple 4 : Synthèse du Tamoxifène (composé de formule (IV)

Après plusieurs cycles de purge à l'argon, un tube de schlenk équippé d'un agitateur magnétique est chargé avec Cul (0,1 mmol, 19 mg), deoxybenzoine (294 mg, 1,5 mmol), Cs₂CO₃ (4 mmol, 1,3 g) et 4-iodophenol (220 mg, 1 mmol). Le tube est purgé à l'argon puis la 2-acetylcyclohexanone (0,1 mmol, 13 µL) est ajoutée à l'aide d'une seringue sous atmosphère d'azote à temperature ambiante puis 1 mL de t-BuOH anhydre est ajouté (1.0 mL). Le tube est scellé sous pression positive d'argon, le mélange réactionnel est ensuite agité et chauffé à 70°C pendant 24 heures. Après refroidissement à température ambiante, le mélange réactionnel est acidifié avec du HCl aqueux (37%) pendant 6 heures. Le mélange réactionnel est ensuite extrait par deux fois avec de l'acétate d'éthyle. Les phases organiques sont récupérées et lavées avec une solution de saumure, séchées sur Na₂SO₄,filtrées et concentrées sous vide. Le produit obtenu est purifié sur chromatographie sur gel de silice (éluant éther de pétrole/acétate d'éthyle 5/5) pour donner le produit A avec un rendement de 82%.

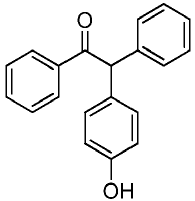

¹H RMN (400 MHz, CDCl₃) δ ppm 8.01-7.84 (m, 2H), 7.51-7.40 (m, 1H), 7.38-7.30 (m, 2H), 7.29-7.21 (m, 2H), 7.20-7.13 (m, 3H), 7.12-7.02 (m, 2H), 6.76-6.66 (m, 2H), 5.90 (s, 1 H).
¹³C RMN (100 MHz, CDCl₃) δ ppm 198.7, 154.8, 139.4, 136.8, 133.1, 131.3, 130.4, 129.1, 129.0, 128.7, 128.6, 127.1, 115.7, Produit A 58.6.

A une solution de produit A (100 mg, 0,347 mmol) dans le THF (3 mL), EtMgBr (350 µL, 1,04 mmol) est ajouté à -78°C et le mélange est agité pendant 5 heures à température ambiante. Le mélange réactionnel résultant est hydrolysé avec de l'eau et extrait par trois fois avec de l'acétate d'éthyle. Les phases organiques obtenues sont combinées, lavées avec une solution de saumure, séchées sur du Na₂SO₄,filtrées puis concentrées sous vide. Le produit ainsi obtenu est dilué avec du méthanol et de l'HCl aqueux (37%) est ajouté. Le mélange réactionnel résultant est agité et mis au reflux pendant 15 heures. Le solvent et l'HCl sont ensuite supprimés par évaporation sous vide. Le produit brut est repris dans de l'acétate d'éthyle et de l'eau est ajoutée. Le mélange est ensuite extrait par trois fois par de l'acétate d'éthyle, les phases organiques sont récupérées, lavées avec une solution de saumure, séchées sur du Na₂SO₄, filtrées puis concentrées sous vide. Le produit obtenu est dilué dans du DMSO (1 mL) et du t-BuOK (155,7 mg, 1,39 mmol) est ajouté. Le mélange réactionnel résultant est chauffé à 50°C pendant 2 heures. Le mélange réactionnel est ensuite hydrolysé avec une solution aqueuse saturée de NH₄Cl à 0°C puis extrait avec de l'acétate d'éthyle et les phases organiques sont récupérées, lavées avec une solution de saumure, séchées sur du Na₂SO₄,filtrées puis concentrées sous vide. Enfin, le produit brut obtenu est dilué dans le DMF (0,5 mL) est ajouté à une solution de NaH (41,6 mg, 1,04 mmol) dans le DMF (0,5mL) à 0°C. Après 30 minutes à 0°C, l'hydrochlorure de 2-Chloro-N,N-diméthyléthylamine(100 mg, 0,69 mmol) est ajouté par portion. Après 3 heures à 50°C, le mélange est hydrolysé à 0°C avec une solution aqueuse saturée de NH₄Cl. Le mélange est ensuite extrait par de l'acétate d'éthyle, les phases organiques sont récupérées, lavées avec une solution de saumure, séchées sur du Na₂SO₄,filtrées puis concentrées sous vide.

Le produit de formule (IV) obtenu est purifié par chromathographie (éluant CHCl₃/MeOH/Et₃N, 95/5/0,1) pour donner un mélange 1/1 de composés de conformation Z et E avec un rendement de 70%.

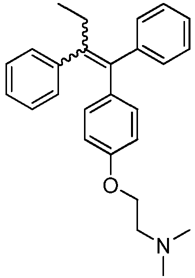

¹H RMN (250 MHz, CDCl₃) δ ppm 7,36-6,73 (m, 24H), 6,69 (d, J = 8,7 Hz, 2H), 6,48 (d, J = 8,7 Hz, 2H), 4,01 (t, J = 5,8 Hz, 2H), 3,85 (t, J = 5,8 Hz, 2H), 2,67 (t, J = 5,8 Hz, 2H), 2,57 (t, J = 5,8 Hz, 2H), 2,43 (q, J = 7,3 Hz, 2H), 2,38 (q, J = 7,3 Hz, 2H), 2,28 (s, 6H), 2,21 (s, 6H), 0,87 (t, J = 7,3 Hz, 3H), 0,85 (t, J = 7,3 Hz, 3H).
¹³C RMN (100 MHz, CDCl₃) δ ppm 157,6, 156,7, 144,3, 143,8, 143,3, 143,0, 142,4, 141,9, 141,6, 141,3, 138,4, 138,2, 136,1, 135,5, 134,7, 131,8, 130,8, 130,5, 130,4, 129,7, 129,5, 129,5, 128,8, 128,1, 127,8, 127,8, 127,3, 126,5, 126,0, 125,6, 114,1, 114,1, 113,4, 65,9, 65,6, 58,4, 58,3, 45,9, 45,9, 29,0, 13,6.

## Revendications

1. Procédé de préparation d'un composé de formule (I) par réaction entre un composé de formule (II) et un composé de formule (III) en présence d'un catalyseur comprenant du cuivre, d'un ligand et d'une base 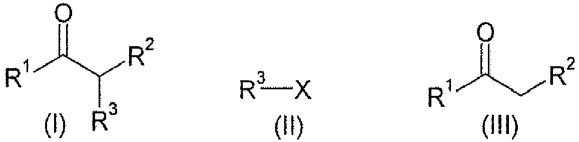 dans lesquelles :
- R¹ représente :
- un atome d'hydrogène ;
- un groupe alkyle comprenant de 1 à 15 atomes de carbone, linéaire ou ramifié,
- un groupe alcényle comprenant de 1 à 15 atomes de carbone, linéaire ou ramifié, et comprenant au moins une double liaison;
- un groupe aryle comprenant de 6 à 10 atomes de carbone, substitué ou non substitué ;
- un groupe hétéroaryle comprenant de 5 à 10 membres dont au moins un hétéroatome, notamment choisi parmi l'atome d'azote ou l'atome d'oxygène, substitué ou non substitué ;
- R² représente :
- un groupe alkyle comprenant de 1 à 15 atomes de carbone, linéaire ou ramifié ;
- un groupe alcényle comprenant de 1 à 15 atomes de carbone, linéaire ou ramifié, et comprenant au moins une double liaison ;
- un groupe aryle comprenant de 6 à 10 atomes de carbone, substitué ou non substitué ;
- un groupe hétéroaryle comprenant de 5 à 10 membres dont au moins un hétéroatome, notamment choisi parmi l'atome d'azote ou l'atome d'oxygène, substitué ou non substitué ;
- R³ représente :
- un groupe vinyle, substitué ou non substitué ;
- un groupe aryle comprenant de 6 à 10 atomes de carbone, substitué ou non substitué ;
- un groupe hétéroaryle comprenant de 5 à 10 membres dont au moins un hétéroatome, notamment choisi parmi l'atome d'azote ou l'atome d'oxygène, substitué ou non substitué ;
- X représente un atome d'halogène choisi parmi le fluor, le chlore, le brome ou l'iode, un groupe tosylate ou un groupe mésylate

2. Procédé selon la revendication 1, pour lequel R³ représente un aryle comprenant de 6 à 10 atomes de carbone non substitué ou substitué par un ou plusieurs substituants choisis parmi :
- un groupe de formule -(O)ₙalkyle comprenant de 1 à 15 atomes de carbone, linéaire ou ramifié, de préférence de 1 à 10 atomes de carbone, par exemple de 1 à 6 atomes de carbone, éventuellement substitué par un ou plusieurs atome d'halogène, de préférence de fluor ; et n représente 0 ou 1 ;
- un groupe alcényle comprenant de 1 à 15 atomes de carbone, linéaire ou ramifié, et comprenant au moins une double liaison;
- un atome d'halogène, notamment choisi parmi le chlore, le fluor, le brome ou l'iode, de préférence le fluor ou le chlore ;
- un groupe de formule CN ;
- un groupe de formule NH₂ ;
- un groupe aryle comprenant de 6 à 10 atomes de carbone, substitué ou non substitué ;
- un groupe de formule (CH₂)ₘ-aryle dans laquelle m représente un nombre entier allant de 1 à 10, de préférence de 1 à 5, et le groupe aryle , substitué ou non substitué, comprend de 6 à 10 atomes de carbone.

3. Procédé selon la revendication 1 ou 2, pour lequel R³ représente un phényle substitué par un ou plusieurs substituants choisis parmi :
- un groupe de formule -(O)ₙalkyle comprenant de 1 à 10 atomes de carbone, linéaire ou ramifié, et n représente 0 ou 1 ;
- un groupe trifluoroalkyle comprenant de 1 à 10 atomes de carbone, linéaire ou ramifié ;
- un atome de fluor ;
- un atome de chlore.

4. Procédé selon la revendication 1, pour lequel le composé de formule (II) est un composé de formule (IIa) 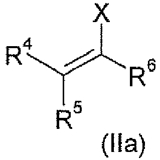 dans laquelle :
- X est tel que défini pour le composé de formule (II), de préférence X représente un atome d'halogène choisi parmi le fluor, le chlore, le brome ou l'iode, de préférence X est l'iode ou le brome ;
- R⁴, R⁵ et R⁶, identiques ou différents, représentent :
- un atome d'hydrogène ;
- un groupe alkyle comprenant de 1 à 15 atomes de carbone, linéaire ou ramifié, pouvant comprendre une ou plusieurs insaturations ;
- un groupe aryle comprenant de 6 à 10 atomes de carbone, substitué ou non substitué ;
- un atome d'halogène choisi parmi le chlore, le brome, le fluor et l'iode.

5. Procédé selon la revendication 1, pour lequel R³ représente un hétéroaryle comprenant de 5 à 10 membres dont au moins un hétéroatome choisi parmi un atome d'azote ou un atome d'oxygène, non substitué ou substitué par un ou plusieurs substituants choisis parmi :
- un groupe de formule -(O)ₙalkyle comprenant de 1 à 15 atomes de carbone, linéaire ou ramifié, de préférence de 1 à 10 atomes de carbone, par exemple de 1 à 6 atomes de carbone, éventuellement substitué par un ou plusieurs atomes d'halogène, de préférence de fluor; et n représente 0 ou 1 ;
- un groupe alcényle comprenant de 1 à 15 atomes de carbone, linéaire ou ramifié, et comprenant au moins une double liaison ;
- un atome d'halogène, notamment choisi parmi le chlore, le fluor, le brome ou l'iode, de préférence le fluor ou le chlore.

6. Procédé selon l'une des revendications 1 à 5, pour lequel dans les composés de formule (I) et (III), R¹ est choisi parmi :
- un atome d'hydrogène ;
- un groupe alkyle comprenant de 1 à 15 atomes de carbone, linéaire ou ramifié ;
- un groupe alcényle comprenant de 1 à 15 atomes de carbone, linéaire ou ramifié, et comprenant au moins une double liaison;
- un groupe aryle comprenant de 6 à 10 atomes de carbone, substitué ou non substitué, notamment par un ou plusieurs substituants choisis parmi :
▪ un atome d'halogène choisi parmi le chlore, le brome, le fluor ou l'iode; de préférence le chlore, le brome ou le fluor, de préférence le chlore;
▪ un groupe de formule -(O)ₚalkyle comprenant de 1 à 15 atomes de carbone, linéaire ou ramifié, de préférence de 1 à 10 atomes de carbone, par exemple de 1 à 6 atomes de carbone et p représente 0 ou 1 ;
▪ un groupe hydroxy ;
▪ un groupe alcényle comprenant de 1 à 15 atomes de carbone, linéaire ou ramifié, et comprenant au moins une double liaison;
▪ un groupe trifluoroalkyle comprenant de 1 à 10 atomes de carbones, linéaire ou ramifié, de préférence trifluorométhyle.

7. Procédé selon l'une des revendications 1 à 6, pour lequel dans les composés de formule (I) et (III) R² est choisi parmi :
- un groupe alkyle comprenant de 1 à 15 atomes de carbone, linéaire ou ramifié;
- un groupe alcényle comprenant de 1 à 15 atomes de carbone, linéaire ou ramifié, et comprenant au moins une double liaison;
- un groupe aryle comprenant de 6 à 10 atomes de carbone, substitué ou non substitué, notamment par un ou plusieurs substituants choisis parmi :
▪ un atome d'halogène choisi parmi le chlore, le brome, le fluor ou l'iode; de préférence le chlore, le brome ou le fluor ;
▪ un groupe hydroxy ;
▪ un groupe de formule -(O)_{q}alkyle comprenant de 1 à 15 atomes de carbone, linéaire ou ramifié, de préférence de 1 à 10 atomes de carbone, par exemple de 1 à 6 atomes de carbone et q représente 0 ou 1 ;
▪ un groupe alcényle comprenant de 1 à 15 atomes de carbone, linéaire ou ramifié, et comprenant au moins une double liaison ;
▪ un groupe trifluoroalkyle comprenant de 1 à 10 atomes de carbones, linéaire ou ramifié, de préférence trifluorométhyle.

8. Procédé selon l'une des revendications 1 à 7, pour lequel X représente l'iode.

9. Procédé selon l'une des revendications 1 à 8 pour lequel le catalyseur est choisi parmi le cuivre métallique, les oxydes de cuivre (I) ou de cuivre (II), les hydroxydes de cuivre (I) ou de cuivre (II), les sels inorganiques ou organiques de cuivre (I) ou de cuivre (II) et les complexes de cuivre (I) ou de cuivre (II) avec des ligands, ou leurs mélanges.

10. Procédé selon l'une des revendications 1 à 9, mis en oeuvre en présence d'un solvant choisi parmi le dioxane et le tert-butanol.

11. Procédé selon l'une des revendications 1 à 10, pour lequel le ligand est choisi parmi :
- les dicétones, notamment de formule (i) 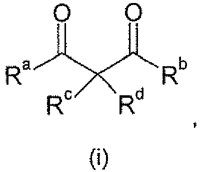 dans laquelle :
▪ R^{a} et R^{b}, identiques ou différents, représentent une chaîne alkyle, linéaire ou ramifiée en C₁ à C₁₀, ou un radical aryle en C₆ à C₁₀ ;
▪ R^{c} et R^{d}, représentent un atome d'hydrogène ; ou
▪ R^{a} ou R^{b}, forme avec le groupe C(O) qui les porte et avec l'un de R^{c} ou R^{d} un carbocycle à 6 membres ; ou
- les diamines de formule (ii)
NR^{e}R^{f}-(CH₂)ᵣ-NR^{g}R^{h} (ii)
dans laquelle:
▪ R^{e}, R^{f}, R^{g}, R^{h}, identiques ou différents, représentent un atome d'hydrogène ou un groupe alkyle en C₁ à C₁₀;
▪ r représente un nombre entier compris allant de 1 à 10 ; ou
- les composés polycycliques aromatiques comprenant au moins deux hétéroatomes, non substitués ou substitués par un ou plusieurs groupes alkyles en C₁ à C₁₀, groupe NO₂, groupe aryle comprenant de 6 à 10 atomes de carbone.

12. Procédé selon l'une des revendications 1 à 11, pour lequel le ligand est choisi parmi les composés de formule :

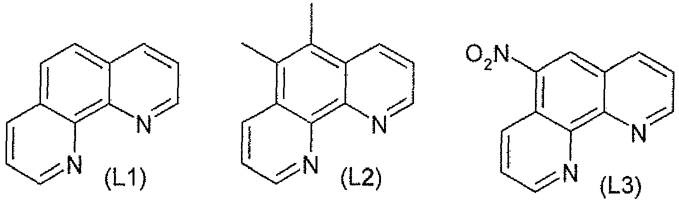

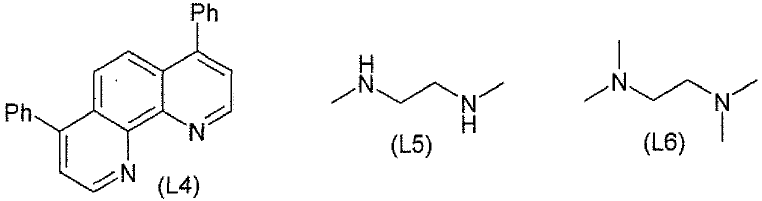

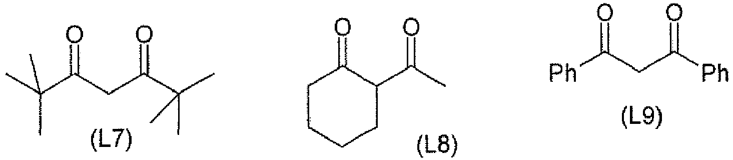

13. Procédé selon l'une des revendications 1 à 12, pour lequel la base est choisie parmi :
- les alcoolates alcalins ou alcalino-terreux de formule (M¹(OR)ₛ) ; et/ou
- les phosphates alcalins ou alcalino-terreux de formule (M¹ₜ(OH)ᵤ(PO₄)ᵥ) ; et/ou
- les carbonates alcalins ou alcalino-terreux de formule (M¹_{w}(CO₃)y),
- ou leurs mélanges
dans lesquelles M¹ représente Li, Na, K, Rb, Cs, Fr, Be, Mg, Ca, Sr, Ba ou Ra ; s représente 1 ou 2 ; R est choisi dans le groupe constitué par les groupes alkyle, benzyle et aryle; t représente un nombre entier allant de 1 à 10 ; u représente un nombre entier allant de 0 à 2 ; v représente un nombre entier allant de 1 à 10 ; w représente un nombre entier allant de 1 à 10 ; y représente un nombre entier allant de 1 à 10.

14. Procédé selon l'une des revendications 1 à 13, mis en oeuvre à une température allant de 50 à 200°C.

15. Procédé selon l'une des revendications 1 à 14 pour lequel la quantité molaire de catalyseur est de 0,001 à 100% par rapport au nombre de moles du composé de formule (II), de préférence de 0,001 à 50%, de manière plus préférée de 0,01 à 25%, par exemple de 1 à 20%.

16. Procédé selon l'une des revendications 1 à 15 pour lequel
- le ratio molaire ligand/catalyseur est de 0,5 à 100. et /ou
- le ratio molaire composé de formule (III)/composé de formule (II) est de 0,5 à 10 ; et/ou
- la quantité molaire de base est de 0,2 à 4 équivalents par rapport à la quantité molaire de composé de formule (II).

17. Procédé de préparation d'un composé de formule (IV) comprenant la mise en oeuvre du procédé selon l'une des revendications 1 à 3 et 6 à 16 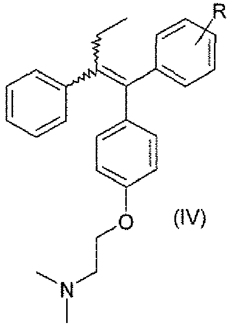 dans laquelle R représente un atome d'hydrogène ou un groupe hydroxy.

18. Procédé selon la revendication 17, comprenant les étapes suivantes :
a) préparation d'un composé de formule (la) comprenant la mise en oeuvre du procédé selon l'une des revendications 1 à 3 et 6 à 16 ; 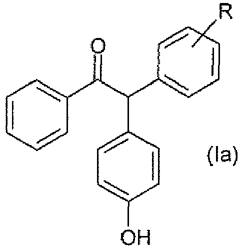 dans laquelle R représente un atome d'hydrogène ou un groupe hydroxy ;
b) réaction du composé de formule (Ia) avec EtMgBr pour obtenir le composé de formule (V) 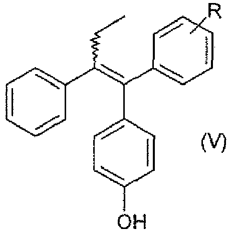
c) réaction du composé de formule (V) avec un composé de formule Y-(CH₂)₂N(Me)₂ dans laquelle Y représente un atome d'halogène pour obtenir le composé de formule (IV) selon la revendication 17.

19. Procédé selon la revendication 18, comprenant les étapes suivantes :
a) préparation d'un composé de formule (Ib) comprenant la mise en oeuvre du procédé de création de liaison C-C selon l'une des revendications 1 à 3 et 6 à 16, en présence d'un composé de formule Y-C₆H₄-O-(CH₂)₂N(Me)₂ dans laquelle Y représente un atome d'halogène, de préférence le chlore ; 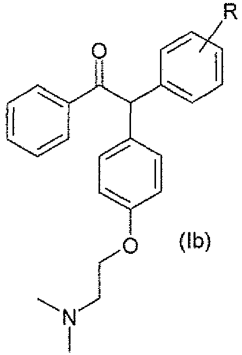 dans laquelle R représente un atome d'hydrogène ou un groupe hydroxy ;
b) réaction du composé de formule (Ib) avec EtMgBr pour obtenir le composé de formule (IV), suivi d'une réaction d'élimination avec de l'acide chlorhydrique puis isomérisation avec le tert-butanoate de potassium dans le DMSO.

## Patentansprüche

1. Verfahren zur Herstellung einer Verbindung der Formel (I) durch Reaktion zwischen einer Verbindung der Formel (II) und einer Verbindung der Formel (III) in Anwesenheit eines Katalysators umfassend Kupfer, eines Liganden und einer Base 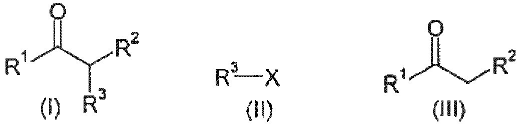 wobei:
- R¹:
- ein Wasserstoffatom;
- eine lineare oder verzweigte Alkylgruppe umfassend 1 bis 15 Kohlenstoffatome;
- eine lineare oder verzweigte Alkenylgruppe umfassend 1 bis 15 Kohlenstoffatome und umfassend mindestens eine Doppelbindung;
- eine substituierte oder unsubstituierte Arylgruppe umfassend 6 bis 10 Kohlenstoffatome;
- eine substituierte oder unsubstituierte Heteroarylgruppe umfassend 5 bis 10 Glieder, von denen mindestens eines ein Heteroatom ist, insbesondere ausgewählt aus Stickstoffatom und Sauerstoffatom
darstellt;
- R²;
- eine lineare oder verzweigte Alkylgruppe umfassend 1 bis 15 Kohlenstoffatome;
- eine lineare oder verzweigte Alkenylgruppe umfassend 1 bis 15 Kohlenstoffatome und umfassend mindestens eine Doppelbindung;
- eine substituierte oder unsubstituierte Arylgruppe umfassend 6 bis 10 Kohlenstoffatome;
- eine substituierte oder unsubstituierte Heteroarylgruppe umfassend 5 bis 10 Glieder, von denen mindestens eines ein Heteroatom ist, insbesondere ausgewählt aus Stickstoffatom und Sauerstoffatom darstellt;
- R³:
- eine substituierte oder unsubstituierte Vinylgruppe;
- eine substituierte oder unsubstituierte Arylgruppe umfassend 6 bis 10 Kohlenstoffatome;
- eine substituierte oder unsubstituierte Heteroarylgruppe umfassend 5 bis 10 Glieder, von denen mindestens eines ein Heteroatom ist, insbesondere ausgewählt aus Stickstoffatom und Sauerstoffatom
darstellt;
- X ein Halogenatom ausgewählt aus Fluor, Chlor, Brom oder Iod, eine Tosylatgruppe oder eine Mesylatgruppe darstellt.

2. Das Verfahren gemäß Anspruch 1, wobei R³ ein Aryl umfassend 6 bis 10 Kohlenstoffatome darstellt, unsubstituiert oder substituiert mit einem oder mehreren Substituenten ausgewählt aus:
- einer linearen oder verzweigten Gruppe der Formel -(O)ₙAlkyl umfassend 1 bis 15 Kohlenstoffatome, vorzugsweise 1 bis 10 Kohlenstoffatome, beispielsweise 1 bis 6 Kohlenstoffatome, wahlweise substituiert mit einem oder mehreren Halogenatomen, vorzugsweise Fluor; und n stellt 0 oder 1 dar;
- einer linearen oder verzweigten Alkenylgruppe umfassend 1 bis 15 Kohlenstoffatome und umfassend mindestens eine Doppelbindung;
- einem Halogenatom, insbesondere ausgewählt aus Chlor, Fluor, Brom oder Iod, vorzugsweise Fluor oder Chlor;
- einer Gruppe der Formel CN;
- einer Gruppe der Formel NH₂;
- einer substituierten oder unsubstituierten Arylgruppe umfassend 6 bis 10 Kohlenstoffatome;
- einer Gruppe der Formel (CH₂)ₘ-Aryl, wobei m eine ganze Zahl von 1 bis 10, vorzugsweise von 1 bis 5 ist und die Arylgruppe, substituiert oder unsubstituiert, 6 bis 10 Kohlenstoffatome umfasst.

3. Das Verfahren gemäß Anspruch 1 oder 2, wobei R³ ein Phenyl darstellt, substituiert mit einem oder mehreren Substituenten ausgewählt aus:
- einer linearen oder verzweigten Gruppe der Formel -(O)ₙAlkyl umfassend 1 bis 10 Kohlenstoffatome, und n stellt 0 oder 1 dar;
- einer linearen oder verzweigten Trifluoroalkylgruppe umfassend 1 bis 10 Kohlenstoffatome;
- einem Fluoratom;
- einem Chloratom.

4. Das Verfahren gemäß Anspruch 1, wobei die Verbindung der Formel (II) eine Verbindung der Formel (IIa) ist 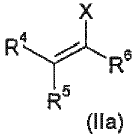 wobei:
- X so wie für die Verbindung der Formel (II) definiert ist, wobei X vorzugsweise ein Halogenatom ausgewählt aus Fluor, Chlor, Brom oder Iod darstellt, wobei X vorzugsweise Iod oder Brom ist;
- R⁴, R⁵ und R⁶, gleich oder verschieden:
- ein Wasserstoffatom;
- eine lineare oder verzweigte Alkylgruppe umfassend 1 bis 15 Kohlenstoffatome, die eine oder mehrere ungesättigte Bindungen umfassen kann;
- eine substituierte oder unsubstituierte Arylgruppe umfassend 6 bis 10 Kohlenstoffatome;
- ein Halogenatom ausgewählt aus Chlor, Brom, Fluor und Iod darstellen.

5. Das Verfahren gemäß Anspruch 1, wobei R³ ein Heteroaryl umfassend 5 bis 10 Glieder darstellt, wobei mindestens eines ein Heteroatom ausgewählt aus Stickstoffatom und Sauerstoffatom ist, nicht substituiert oder substituiert mit einem oder mehreren Substituenten ausgewählt aus:
- einer linearen oder verzweigten Gruppe der Formel -(O)ₙAlkyl umfassend 1 bis 15 Kohlenstoffatome, vorzugsweise 1 bis 10 Kohlenstoffatome, beispielsweise 1 bis 6 Kohlenstoffatome, wahlweise substituiert mit einem oder mehreren Halogenatomen, vorzugsweise Fluor; und n stellt 0 oder 1 dar;
- einer linearen oder verzweigten Alkenylgruppe umfassend 1 bis 15 Kohlenstoffatome und umfassend mindestens eine Doppelbindung;
- einem Halogenatom, insbesondere ausgewählt aus Chlor, Fluor, Brom oder Iod, vorzugsweise Fluor oder Chlor.

6. Das Verfahren gemäß einem der Ansprüche 1 bis 5, wobei, in den Verbindungen der Formel (I) und (III), R¹ ausgewählt ist aus:
- einem Wasserstoffatom;
- einer linearen oder verzweigten Alkylgruppe umfassend 1 bis 15 Kohlenstoffatome;
- einer linearen oder verzweigten Alkenylgruppe umfassend 1 bis 15 Kohlenstoffatome und umfassend mindestens eine Doppelbindung;
- einer Arylgruppe umfassend 6 bis 10 Kohlenstoffatome, substituiert oder unsubstituiert, insbesondere mit einem oder mehreren Substituenten ausgewählt aus:
▪ einem Halogenatom ausgewählt aus Chlor, Brom, Fluor oder Iod; vorzugsweise Chlor, Brom oder Fluor, vorzugsweise Chlor;
▪ einer linearen oder verzweigten Gruppe der Formel -(O)ₚAlkyl umfassend 1 bis 15 Kohlenstoffatome, vorzugsweise 1 bis 10 Kohlenstoffatome, beispielsweise 1 bis 6 Kohlenstoffatome und p stellt 0 oder 1 dar;
▪ einer Hydroxygruppe;
▪ einer linearen oder verzweigten Alkenylgruppe umfassend 1 bis 15 Kohlenstoffatome und umfassend mindestens eine Doppelbindung;
▪ einer linearen oder verzweigten Trifluoroalkylgruppe umfassend 1 bis 10 Kohlenstoffatome, vorzugsweise Trifluoromethyl.

7. Das Verfahren gemäß einem der Ansprüche 1 bis 6, wobei, in den Verbindungen der Formel (I) und (III), R² ausgewählt ist aus:
- einer linearen oder verzweigten Alkylgruppe umfassend 1 bis 15 Kohlenstoffatome;
- einer linearen oder verzweigten Alkenylgruppe umfassend 1 bis 15 Kohlenstoffatome und umfassend mindestens eine Doppelbindung;
- einer Arylgruppe umfassend 6 bis 10 Kohlenstoffatome, substituiert oder unsubstituiert, insbesondere mit einem oder mehreren Substituenten ausgewählt aus:
▪ einem Halogenatom ausgewählt aus Chlor, Brom, Fluor oder Iod; vorzugsweise Chlor, Brom oder Fluor;
▪ einer Hydroxygruppe
▪ einer linearen oder verzweigten Gruppe der Formel -(O)_{q}Alkyl umfassend 1 bis 15 Kohlenstoffatome, vorzugsweise 1 bis 10 Kohlenstoffatome, beispielsweise 1 bis 6 Kohlenstoffatome und q stellt 0 oder 1 dar;
▪ einer linearen oder verzweigten Alkenylgruppe umfassend 1 bis 15 Kohlenstoffatome und umfassend mindestens eine Doppelbindung;
▪ einer linearen oder verzweigten Trifluoroalkylgruppe umfassend 1 bis 10 Kohlenstoffatome, vorzugsweise Trifluoromethyl.

8. Das Verfahren gemäß einem der Ansprüche 1 bis 7, wobei X Iod darstellt.

9. Das Verfahren gemäß einem der Ansprüche 1 bis 8, wobei der Katalysator ausgewählt ist aus metallischem Kupfer, Kupfer(I)- oder Kupfer(II)-Oxiden, Kupfer(I)- oder Kupfer(II)-Hydroxiden, anorganischen oder organischen Kupfer(I)-oder Kupfer(II)-Salzen und Kupfer(I)- oder Kupfer(II)-Komplexen mit Liganden, oder deren Mischungen.

10. Das Verfahren gemäß einem der Ansprüche 1 bis 9, umgesetzt in Anwesenheit eines Lösemittels ausgewählt aus Dioxan und tert-Butanol.

11. Das Verfahren gemäß einem der Ansprüche 1 bis 10, wobei der Ligand ausgewählt ist aus:
- Diketonen, insbesondere der Formel (i) 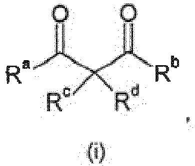 wobei:
▪ R^{a} und R^{b}, gleich oder verschieden, eine lineare oder verzweigte Alkylkette mit C₁ bis C₁₀ oder ein Arylradikal mit C₆ bis C₁₀ darstellen;
▪ R^{c} und R^{d} ein Wasserstoffatom darstellen; oder
▪ R^{a} und R^{b} bilden mit der C(O)-Gruppe, die sie tragen, und mit einem von R^{c} und R^{d} einen 6-gliedrigen Carbocyclus; oder
- Diaminen der Formel (ii)
NR^{e}R^{f}-(CH₂)ᵣ-NR^{g}R^{h} (ii)
wobei:
▪ R^{e}, R^{f}, R^{g}, R^{h}, gleich oder verschieden, ein Wasserstoffatom oder eine Alkylgruppe mit C₁ bis C₁₀ darstellen;
▪ r eine ganze Zahl zwischen 1 bis 10 darstellt; oder
- polycyclischen, aromatischen Verbindungen umfassend mindestens zwei Heteroatome, unsubstituiert oder substituiert mit einer oder mehreren Alkylgruppen mit C₁ bis C₁₀, NO₂-Gruppe, Arylgruppe umfassend 6 bis 10 Kohlenstoffatome.

12. Das Verfahren gemäß einem der Ansprüche 1 bis 11, wobei der Ligand ausgewählt ist aus den Verbindungen der Formel:

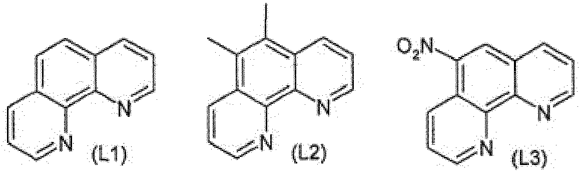

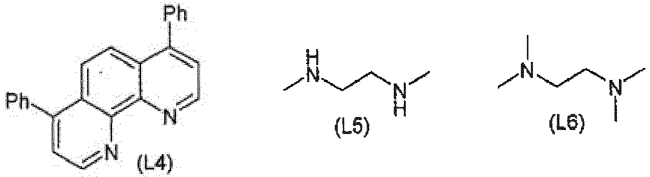

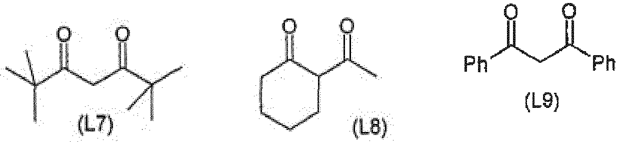

13. Das Verfahren gemäß einem der Ansprüche 1 bis 12, wobei die Base ausgewählt ist aus:
- Alkali- oder Erdalkali-Alkoholaten der Formel (M¹(OR)ₛ); und/oder
- Alkali- oder Erdalkali-Phosphaten der Formel (M¹₁(OH)ᵤ(PO₄)ᵥ); und/oder
- Alkali- oder Erdalkali-Carbonaten der Formel (M'_{w}(CO₃)_{y}),
- oder deren Mischungen,
wobei M¹ Li, Na, K, Rb, Cs, Fr, Be, Mg, Ca, Sr, Ba oder Ra darstellt; s 1 oder 2 darstellt; R ausgewählt ist aus der Gruppe bestehend aus Alkyl-, Benzyl- und Arylgruppen; t eine ganze Zahl von 1 bis 10 darstellt; u eine ganze Zahl von 0 bis 2 darstellt; v eine ganze Zahl von 1 bis 10 darstellt; w eine ganze Zahl von 1 bis 10 darstellt; y eine ganze Zahl von 1 bis 10 darstellt.

14. Das Verfähren gemäß einem der Ansprüche 1 bis 13, umgesetzt bei einer Temperatur von 50 bis 200 °C.

15. Das Verfahren gemäß einem der Ansprüche 1 bis 14, wobei die molare Menge an des Katalysators 0,001 bis 100 % ist, bezogen auf die Anzahl an Molen der Verbindung der Formel (II), vorzugsweise 0,001 bis 50 %, noch bevorzugter 0,01 bis 25 %, beispielsweise 1 bis 20 %.

16. Das Verfahren gemäß einem der Ansprüche 1 bis 15, wobei
- das molare Verhältnis Ligand/Katalysator 0,5 bis 100 ist; und/oder
- das molare Verhältnis Verbindung der Formel (III)/Verbindung der Formel (II) 0,5 bis 10 ist; und/oder
- die molare Menge der Base 0,2 bis 4 Äquivalente ist, bezogen auf die molare Menge der Verbindung der Formel (II).

17. Verfahren zur Herstellung einer Verbindung der Formel (IV), umfassend das Umsetzen des Verfahren gemäß einem der Ansprüche 1 bis 3 und 6 bis 16 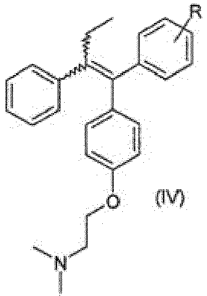 wobei R ein Wasserstoffatorn oder eine Hydroxygruppe darstellt.

18. Das Verfahren gemäß Anspruch 17, umfassend die folgenden Schritte:
a) Herstellen einer Verbindung der Formel (Ia) umfassend das Umsetzen des Verfahrens gemäß einem der Ansprüche 1 bis 3 und 6 bis 16; 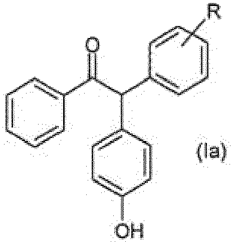 wobei R ein Wasserstoffatom oder eine Hydroxygruppe darstellt;
b) Reagieren-lassen der Verbindung der Formel (Ia) mit EtMgBr, um die Verbindung der Formel (V) zu erhalten; 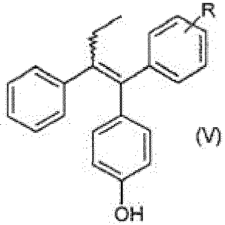
c) Reagieren-lassen der Verbindung der Formel (V) mit einer Verbindung der Formel Y-(CH₂)₂N(Me)₂, wobei Y ein Halogenatom darstellt, um die Verbindung der Formel (IV) gemäß Anspruch 17 zu erhalten.

19. Das Verfahren gemäß Anspruch 18, umfassend die folgenden Schritte:
a) Herstellen einer Verbindung der Formel (Ib) umfassend das Umsetzen des Verfahrens der Bildung der C-C-Bindung gemäß einem der Ansprüche 1 bis 3 und 6 bis 16 in Anwesenheit einer Verbindung der Formel Y-C₆H₄-O-(CH₂)₂N(Me)₂, wobei Y ein Halogenatom darstellt, vorzugsweise Chlor; 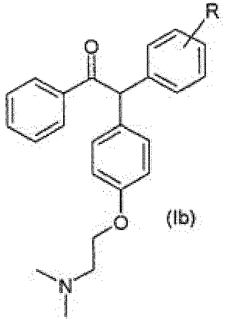 wobei R ein Wasserstoffatom oder eine Hydroxygruppe darstellt;
b) Reagieren-lassen der Verbindung der Formel (Ib) mit EtMgBr, um die Verbindung der Formel (IV) zu erhalten, gefolgt von einer Eliminierungsreaktion mit Salzsäure, hiernach Isomerisierung mit Kalium-tert-Butyrat in DMSO.

## Claims

1. A process for preparing a compound of formula (I) by reaction between a compound of formula (II) and a compound of formula (III) in the presence of a copper-containing catalyst , a ligand and a base 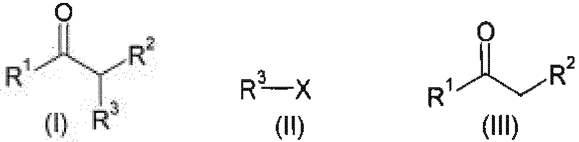 where:
- R¹ is:
- a hydrogen atom;
- a straight-chain or branched alkyl group having 1 to 15 carbon atoms;
- a straight-chain or branched alkenyl group having 1 to 15 carbon atoms and comprising at least one double bond;
- a substituted or unsubstituted aryl group having 6 to 10 carbon atoms;
- a substituted or unsubstituted heteroaryl group having 5 to 10 members including at least one heteroatom selected in particular from among a nitrogen atom or oxygen atom;
- R² is:
- a straight-chain or branched alkyl group having 1 to 15 carbon atoms:
- a straight-chain or branched alkenyl group having 1 to 15 carbon atoms and comprising at least one double bond;
- a substituted or unsubstituted aryl group having 6 to 10 carbon atoms;
- a substituted or unsubstituted heteroaryl group having 5 to 10 members including at least one heteroatom selected in particular from among a nitrogen atom or oxygen atom;
- R³ is:
- a substituted or unsubstituted vinyl group;
- a substituted or unsubstituted aryl group having 6 to 10 carbon atoms;
- a substituted or unsubstituted heteroaryl group having 5 to 10 members including at least one heteroatom selected in particular from among a nitrogen atom or oxygen atom;
- X is a halogen atom selected from among fluorine, chlorine, bromine or iodine, a tosylate group or mesylate group.

2. The process according to claim 1 wherein R³ represents an aryl having 6 to 10 carbon atoms, unsubstituted or substituted by one or more substituents selected from among:
- a straight-chain or branched group of formula -(O)ₙalkyl having 1 to 15 carbon atoms, preferably 1 to 10 carbon atoms e.g. 1 to 6 carbon atoms, optionally substituted by one or more halogen atoms, preferably fluorine; and n is 0 or 1;
- a straight-chain or branched alkenyl group having 1 to 15 carbon atoms and comprising at least one double bond;
- a halogen atom selected in particular from among chlorine, fluorine, bromine or iodine, preferably fluorine or chlorine;
- a group of formula CN;
- a group of formula NH₂;
- a substituted or unsubstituted aryl group having 6 to 10 carbon atoms;
- a group of formula (CH₂)ₘ-aryl wherein m is an integer ranging from 1 to 10, preferably 1 to 5, and the aryl group, substituted or unsubstituted, comprises 6 to 10 carbon atoms.

3. The process according to claim 1 or 2 wherein R³ represents a phenyl substituted by one or more substituents selected from among:
- a straight-chain or branched group of formula -(O)ₙalkyl having 1 to 10 carbon atoms, and n is 0 or 1;
- a straight-chain or branched trifluoroalkyle group having 1 to 10 carbon atoms;
- a fluorine atom;
- a chlorine atom.

4. The process according to claim 1 wherein the formula (II) compound is a compound of formula (IIa) 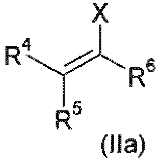 where:
- X is such as defined for the formula (II) compound, preferably X is a halogen atom selected from among fluorine, chlorine, bromine or iodine, preferably X is iodine or bromine;
- R⁴, R⁵ and R⁶, the same or different, represent:
- a hydrogen atom;
- a straight-chain or branched alkyl group having 1 to 15 carbon atoms, possibly comprising one or more unsaturations;
- a substituted or unsubstituted aryl group having 6 to 10 carbon atoms;
- a halogen atom selected from among chlorine, bromine, fluorine and iodine.

5. The process according to claim 1 wherein R³ represents a heteroaryl having 5 to 10 members including at least one heteroatom selected from among a nitrogen atom or oxygen atom, unsubstituted or substituted by one or more substituents selected from among:
- a straight-chain or branched group of formula -(O)ₙalkyl having 1 to 15 carbon atoms, preferably 1 to 10 carbon atoms, e.g. 1 to 6 carbon atoms, optionally substituted by one or more halogen atoms, preferably fluorine; and n is 0 or 1;
- a straight-chain or branched alkenyl group having 1 to 15 carbon atoms and comprising at least one double bond;
- a halogen atom selected in particular from among chlorine, fluorine, bromine or iodine, preferably fluorine or chlorine.

6. The process according to one of claims 1 to 5 wherein for the compounds of formula (I) and (III), R¹ is selected from among:
- a hydrogen atom;
- a straight-chain or branched alkyl group having 1 to 15 carbon atoms;
- a straight-chain or branched alkenyl group having 1 to 15 carbon atoms and comprising at least one double bond;
- an aryl group having 6 to 10 carbon atoms, unsubstituted or substituted in particular by one or more substituents selected from among:
▪ a halogen atom selected from among chlorine, bromine, fluorine or iodine; preferably chlorine, bromine or fluorine, preferably chlorine;
▪ a straight-chain or branched group of formula -(O)ₚalkyl having 1 to 15 carbon atoms, preferably 1 to 10 carbon atoms e.g. 1 to 6 carbon atoms, and p is 0 or 1;
▪ a hydroxy group,
▪ a straight-chain or branched alkenyl group having 1 to 15 carbon atoms and comprising at least one double bond;
▪ a straight-chain or branched trifluoroalkyl preferably trifluoromethyl group having 1 to 10 carbon atoms.

7. The process according to one of claims 1 to 6 wherein for the compounds of formula (I) and (III) R² is selected from among:
- a straight-chain or branched alkyl group having 1 to 15 carbon atoms;
- a straight-chain or branched alkenyl group having 1 to 15 carbon atoms and comprising at least one double bond;
- an aryl group having 6 to 10 carbon atoms unsubstituted or substituted in particular by one or more substituents selected from among:
▪ a halogen atom selected from among chlorine, bromine, fluorine or iodine; preferably chorine, bromine or fluorine;
▪ a hydroxy group;
▪ a straight-chain or branched group of formula -(O)_{q}alkyl having 1 to 15 carbon atoms, preferably 1 to 10 carbon atoms e.g. 1 to 6 carbon atoms, and q is 0 or 1;
▪ a straight-chain or branched alkenyl group having 1 to 15 carbon atoms and comprising at least one double bond;
▪ a straight-chain or branched trifluoroalkyl preferably trifluoromethyl group having 1 to 10 carbon atoms.

8. The process according to one of claims 1 to 7 wherein X represents iodine.

9. The process according to one of claims 1 to 8 wherein the catalyst is selected from among metallic copper, copper(I) or copper(II) oxides, copper(I) or copper(II) hydroxides, the inorganic or organic salts of copper(I) or copper(II) and the complexes of copper(I) or copper(II) with ligands, or the mixtures thereof

10. The process according to one of claims 1 to 9 implemented in the presence of a solvent selected from among dioxane and tert-butanol.

11. The process according to one of claims 1 to 10 wherein the ligand is selected from among:
- diketones, in particular of formula (i): 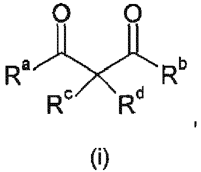 where:
▪ R^{a} and R^{b}, the same or different, represent a straight-chain or branched C₁ to C₁₀ alkyl chain, or C₆ to C₁₀ aryl radical;
▪ R^{c} and R^{d} are a hydrogen atom; or
▪ R^{a} or R^{b}, together with their carrier group C(O) and with one of R^{c} or R^{d}, forms a 6-membered carbocycle; or
- diamines of formula (ii)
NR^{e}R^{f}-(CH₂)ᵣ-NR^{g}R^{h} (ii)
where:
▪ R^{e}, R^{f}, R^{g}, R^{h}, the same or different, represent a hydrogen atom or C₁ to C₁₀ alkyl group;
▪ r is an integer ranging from 1 to 10; or
- aromatic polycyclic compounds having at least two heteroatoms, unsubstituted or substituted by one or more C₁ to C₁₀ alkyl groups, NO₂ group, aryl group having 6 to 10 carbon atoms.

12. The process according to one of claims 1 to 11 wherein the ligand is selected from among the compounds of formula:

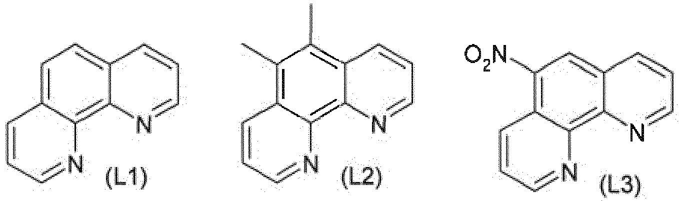

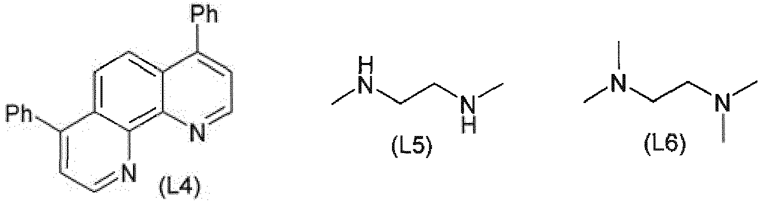

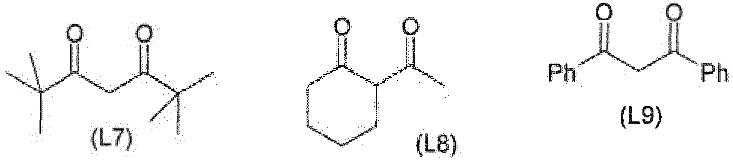

13. The process according to one of claims 1 to 12 wherein the base is selected from among:
- alkaline or alkaline-earth alcoholates of formula (M¹(OR)ₛ): and/or
- alkaline or alkaline-earth phosphates of formula (M¹ₜ(OH)ᵤ(PO₄)ᵥ); and/or
- alkaline or alkaline-earth carbonates of formula (M¹_{w}(CO₃)_{y}),
- or the mixtures thereof
wherein M¹ is Li, Na, K, Rb, Cs, Fr, Be, Mg, Ca, Sr, Ba or Ra; s is 1 or 2; R is selected from the group formed by the alkyl, benzyl and aryl groups; t is an integer ranging from 1 to 10; u is an integer ranging from 0 to 2; v is an integer ranging from 1 to 10; w is an integer ranging from 1 to 10; y is an integer ranging from 1 to 10.

14. The process according to one of claims 1 to 13 implemented at a temperature ranging from 50 to 200°C.

15. The process according to one of claims 1 to 14 wherein the molar quantity of catalyst is 0.001 to 100 % relative to the number of moles of the formula (II) compound, preferably 0.001 to 50 %, more preferably 0.01 to 25 %, e.g. from 1 to 20 %.

16. The process according to one of claims 1 to 15 wherein:
- the ligand/catalyst molar ratio is 0.5 to 100; and/or
- the molar ratio of formula (III) compound/formula (II) compound is 0.5 to 10; and/or
- the molar quantity of base is 0.2 to 4 equivalents relative to the molar quantity of formula (II) compound.

17. A process for preparing a compound of formula (IV) comprising the implementing of the process according to one of claims 1 to 3 and 6 to 16: 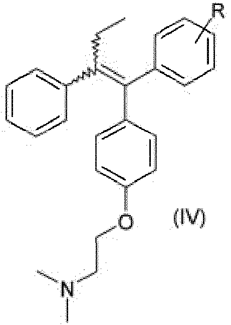 where R is a hydrogen atom or hydroxy group.

18. The process according to claim 17 comprising the following steps:
a) preparing a compound of formula (Ia) comprising the implementing of the process according to one of claims 1 to 3 and 6 to 16: 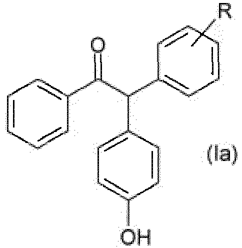 where R is a hydrogen atom or hydroxy group;
b) reacting the formula (Ia) compound with EtMgBr to obtain the compound of formula (V): 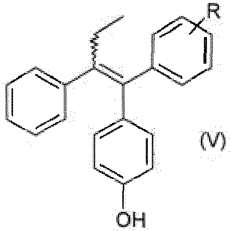
c) reacting the formula (V) compound with a compound of formula Y-(CH₂)₂N(Me)₂ where Y is a halogen atom, to obtain the compound of formula (IV) according to claim 17.

19. The process according to claim 18, comprising the following steps:
a) preparing a compound of formula (Ib) comprising the implementing of the process to create a C-C bond according to one of claims 1 to 3 and 6 to 16, in the presence of a compound of formula Y-C₆H₄-O-(CH₂)₂N(Me)₂ where Y is a halogen atom, preferably chlorine; 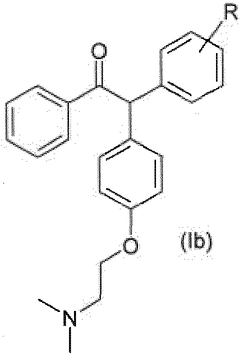 where R is a hydrogen atom or hydroxy group;
b) reacting the formula (Ib) compound with EtMgBr to obtain the compound of formula (IV), followed by an elimination reaction with hydrochloric acid and then isomerisation with potassium tert-butanoate in DMSO.
